(19) Europäisches Patentamt
European Patent Office
Office européen des brevets

(11) **EP 4 458 799 B1**

(12) **EUROPEAN PATENT SPECIFICATION**

(45) Date of publication and mention of the grant of the patent:
**25.03.2026 Bulletin 2026/13**

(21) Application number: **23863403.4**

(22) Date of filing: **30.08.2023**

(51) International Patent Classification (IPC):
***C07C 51/09*** *(2006.01)* ***C07C 63/26*** *(2006.01)*
***C07C 67/03*** *(2006.01)* ***C07C 67/56*** *(2006.01)*
***C07C 69/82*** *(2006.01)* ***C08J 11/10*** *(2006.01)*
***C08G 63/183*** *(2006.01)* ***C07C 67/60*** *(2006.01)*

(52) Cooperative Patent Classification (CPC):
(C-Sets available)
**C07C 67/03; C07C 51/09; C07C 63/26; C07C 67/60; C07C 69/82; C08G 63/183; C08J 11/14;** C08J 2367/02 (Cont.)

(86) International application number:
**PCT/KR2023/012903**

(87) International publication number:
**WO 2024/053927 (14.03.2024 Gazette 2024/11)**

(54) **METHOD FOR PRODUCING TEREPHTHALIC ACID AND TEREPHTHALIC ACID PRODUCED THEREFROM**

VERFAHREN ZUR HERSTELLUNG VON TEREPHTHALSÄURE UND DARAUS HERGESTELLTE TEREPHTHALSÄURE

PROCÉDÉ DE PRODUCTION D'ACIDE TÉRÉPHTALIQUE ET ACIDE TÉRÉPHTALIQUE AINSI PRODUIT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **05.09.2022 KR 20220112205**
**28.06.2023 KR 20230083807**

(43) Date of publication of application:
**06.11.2024 Bulletin 2024/45**

(73) Proprietor: **SK Chemicals Co., Ltd.**
**Seongnam-si, Gyeonggi-do 13494 (KR)**

(72) Inventors:
- **CHANG, Yu-Mi**
**Seongnam-si, Gyeonggi-do 13494 (KR)**
- **KANG, Ju-Sik**
**Seongnam-si, Gyeonggi-do 13494 (KR)**
- **PARK, Jeong Ho**
**Seongnam-si, Gyeonggi-do 13494 (KR)**
- **CHAE, Hee-Il**
**Seongnam-si, Gyeonggi-do 13494 (KR)**

(74) Representative: **Dehns**
**10 Old Bailey**
**London EC4M 7NG (GB)**

(56) References cited:
**CN-A- 104 722 248** **JP-A- 2006 083 125**
**JP-A- 2006 083 125** **JP-A- H0 717 901**
**JP-A- H06 157 402** **JP-A- H06 240 046**
**KR-A- 20200 046 198** **US-A- 5 095 145**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 51/09, C07C 63/26;**
**C07C 67/03, C07C 69/82**

## Description

### Technical Field

The present invention relates to a process for preparing terephthalic acid in an environmentally friendly manner using waste polyester and to recycled terephthalic acid prepared thereby.

### Background Art

Polyester is widely used as a material for beverage-filling containers, packaging films, audio and video films, and the like, or an industrial material such as medical fibers and tire cords, by virtue of its excellent mechanical strength, thermal resistance, transparency, and gas barrier properties. In particular, polyester sheets or plates have good transparency and excellent mechanical strength, so that they are widely used as raw materials for cases, boxes, partitions, shelves, panels, packaging materials, building materials, interior and exterior materials, and the like.

As waste of plastics such as polyester is generated globally at an unmanageable level every year, there is increasing interest in recycling waste polyester or regeneration processes using waste polyester. In addition, countries around the world have prepared regulations and plans for recycling waste plastic resources, including waste polyester. For example, regulations that require a recycled resin to be used at a certain ratio or more in packaging materials used in various fields are being discussed.

In particular, since polyethylene terephthalate (PET) has excellent properties in terms of thermal resistance, processability, transparency, and non-toxicity, it is widely used to manufacture a wide range of products such as films, fibers, bottles, and containers. However, most of them are landfilled or incinerated after use; thus, research on recycling or regeneration processes using them is continuing.

For example, Korean Laid-open Patent Publication No. 1997-0042469 discloses a technology for preparing terephthalic acid by hydrolyzing waste polyethylene terephthalate with an aqueous alkaline solution to obtain a slurry of alkali metal and alkali earth metal salts of terephthalic acid and neutralizing it with an acid. A terephthalic acid salt, rather than terephthalic acid, is produced as a result of the hydrolysis reaction, and a neutralization step by adding acid is necessary to convert it to terephthalic acid. There is a problem in that environmental pollutants are generated from the by-products formed as a result, or a large amount of acid treatment waste solutions is generated.

[Prior Art Document]

[Patent Document]

(Patent Document 1) Korean Laid-open Patent Publication No. 1997-0042469

JP 2006 083125 A discloses a method for producing terephthalic acid.

### Disclosure of Invention

### Technical Problem

Accordingly, **the** present invention aims to provide a process for preparing terephthalic acid in an environmentally friendly manner by alcoholysis and hydrolysis of waste polyester using a specific alcohol, and recycled terephthalic acid prepared thereby.

### Solution to Problem

The process for preparing terephthalic acid according to an embodiment of the present invention comprises (1) subjecting waste polyester to alcoholysis with an alcohol having 4 or more carbon atoms to prepare a liquid composition comprising a compound represented by Formula 1; and (2) subjecting the liquid composition to hydrolysis.

[Formula 1]

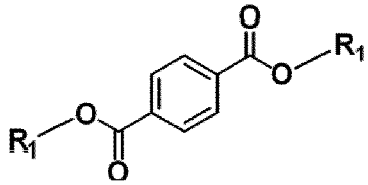

In Formula 1, $R_1$ is an alkyl group having 4 or more carbon atoms.

Recycled terephthalic acid is prepared according to the above process for preparing terephthalic acid and has a total metal content of less than 100 ppm when measured by inductively coupled plasma atomic emission spectroscopy (ICP-AES).

Also disclosed herein is a polyester resin that comprises the recycled terephthalic acid.

## Advantageous Effects of Invention

In the process for preparing terephthalic acid according to an embodiment of the present invention, waste polyester is subjected to alcoholysis with an alcohol having 4 or more carbon atoms to prepare a liquid composition comprising a compound represented by Formula 1, which is then subjected to hydrolysis with water. Thus, terephthalic acid can be prepared in an environmentally friendly manner, and processability can be enhanced along with reduced process costs.

Specifically, in the process for preparing terephthalic acid according to an embodiment of the present invention, waste polyester is subjected to alcoholysis with a specific alcohol, more specifically an alcohol having 4 or more carbon atoms, to prepare a liquid composition comprising a compound represented by Formula 1, which is then subjected to hydrolysis with water. In the process for preparing terephthalic acid according to an embodiment of the present invention, solid terephthalic acid can be directly produced without additional steps, unlike the conventional process in which a terephthalic acid salt is produced from waste polyester, and an additional step of neutralizing it is performed. Thus, not only can the present process be easily operated, but process costs can also be reduced, resulting in excellent processability.

In addition, there has been a problem in that acids such as sulfuric acid or hydrochloric acid used to neutralize terephthalic acid salts generate environmental pollutants such as $Na_2SO_4$ and NaCl as by-products or a large amount of acid treatment waste solutions. In contrast, in the process for preparing terephthalic acid according to an embodiment of the present invention, unlike the conventional process, solid terephthalic acid can be directly produced without performing an additional neutralization step, making it environmentally friendly.

In addition, in the process for preparing terephthalic acid according to an embodiment of the present invention, an intermediate, that is, a hydrolysis reactant, is prepared in a liquid form; thus, insoluble impurities such as metal catalysts and coloring pigments, and additives such as soluble colorants that may be contained in waste polyester can be easily removed. Thus, the purity and yield of terephthalic acid produced through a simple process can be further enhanced.

## Best Mode for Carrying out the Invention

Hereinafter, the present invention will be described in detail. The present invention is not limited to the disclosures given below, but it may be modified into various forms as long as the gist of the invention is not changed.

Throughout the present specification, when a part is referred to as "comprising" an element, it is understood that other elements may be comprised, rather than other elements are excluded, unless specifically stated otherwise.

All numbers and expressions related to the quantities of components, reaction conditions, and the like used herein are to be understood as being modified by the term "about," unless otherwise indicated.

Throughout the present specification, the terms first, second, and the like are used to describe various components. But the components should not be limited by the terms. The terms are used only for the purpose of distinguishing one component from another.

The process for preparing terephthalic acid according to an embodiment of the present invention comprises (1) subjecting waste polyester to alcoholysis with an alcohol having 4 or more carbon atoms to prepare a liquid composition comprising a compound represented by Formula 1; and (2) subjecting the liquid composition to hydrolysis.

[Formula 1]

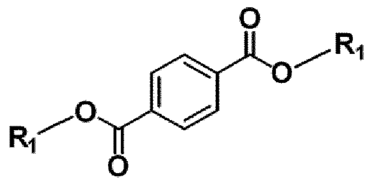

In Formula 1, $R_1$ is an alkyl group having 4 or more carbon atoms.

In the conventional methods for preparing terephthalic acid from waste polyester, solid terephthalic acid is directly obtained using an acid catalyst, or dimethyl terephthalate (DMT) or bis(2-hydroxyethyl) terephthalate (BHET) is produced as an intermediate through methanolysis or glycolysis, which is then converted to terephthalic acid. In such an event, terephthalic acid, dimethyl terephthalate, and bis(2-hydroxyethyl) terephthalate are all solids at room temperature or at relatively high temperatures.

When solid terephthalic acid is directly obtained using an acid catalyst, terephthalic acid is precipitated as a solid immediately after being formed, making it difficult to remove insoluble impurities or additives such as colorants and pigments that may be contained in waste polyester during the manufacturing process. Thus, there are limits to enhancing purity or yield. For this reason, a method of preparing a terephthalic acid salt from waste polyester and then neutralizing it to obtain solid terephthalic acid is also used. In such an event, although the purity and yield of terephthalic acid can be enhanced by removing insoluble impurities or the like, there is a problem in that environmental pollutants such as $Na_2SO_4$ and NaCl as by-products or a large amount of acid treatment waste solutions are generated in the process of neutralizing the terephthalic acid salt.

In addition, when dimethyl terephthalate is prepared as an intermediate, a large amount of methanol must be used to produce dimethyl terephthalate. Thus, very high-pressure conditions are generated due to the methanol used, and an additional heating and high-pressure process to convert the dimethyl terephthalate into a liquid phase is required to remove insoluble impurities, making the processability low. In addition, when bis(2-hydroxyethyl) terephthalate is prepared as an intermediate, it is difficult to separate and recover ethylene glycol, which is generated as a by-product in the manufacturing process; thus, it is undesirable in terms of processability and process cost.

In addition, an environmentally friendly method of recycling waste polyester is used by adding metal salts to waste polyester and directly hydrolyzing it with water. However, processability is low in that very high-temperature conditions of 300°C or higher are required, and the reaction apparatus must also have high-pressure resistance.

In contrast, in the process for preparing terephthalic acid according to an embodiment of the present invention, an intermediate, that is, a hydrolysis reactant, is prepared in a liquid form; thus, insoluble impurities and additives such as colorants and pigments that may be contained in waste polyester can be easily removed. Thus, the purity and yield of terephthalic acid produced can be enhanced.

In addition, since solid terephthalic acid can be directly produced without performing an additional neutralization step, unlike the conventional process, processability is excellent, along with environmental friendliness. Further, not only is it easy to separate and recover ethylene glycol, which may be formed as a by-product during the preparation process, but the alcohol with 4 or more carbon atoms used in the alcoholysis reaction can also be readily separated and reused, resulting in excellent process cost reduction effect. In addition, processability and economic efficiency can be further enhanced in that purification and transfer processes other than reaction can be carried out at room temperature or low temperatures.

For example, in the process for preparing terephthalic acid according to an embodiment of the present invention, first, waste polyester, an alcohol with 4 or more carbon atoms, and a very minute amount of an alcoholysis catalyst are charged to a first high-pressure reactor, followed by an alcoholysis reaction. Ethylene glycol, which is a by-product formed as the alcoholysis reaction is carried out, and unreacted alcohol (alcohol present in excess) can be recovered through a separate fractional distillation apparatus upon completion of the reaction and reused.

In addition, ethylene glycol formed during the reaction and unreacted alcohol may be discharged in the form of a gaseous mixture in real-time during the reaction, which is then condensed using an external cooling device and recovered. In such an event, an alcohol may be continuously fed to the high-pressure reactor at the same volume and feeding rate as the volume and discharge rate of the discharged gaseous mixture. The unreacted alcohol can be separated from the discharged gaseous mixture through a simple process such as fractional distillation or layer separation. The unreacted alcohol thus separated can be fed to the first high-pressure reactor again, and ethylene glycol can be recovered therefrom.

Thereafter, the liquid alcoholysis product obtained through the alcoholysis reaction can be purified by cooling, adsorption, and filtration. The purified alcoholysis reaction composition, together with water, is fed to a second high-pressure reactor to carry out a hydrolysis reaction, and a slurry-type solution is then obtained and filtered to obtain solid terephthalic acid. In such an event, a small amount of a hydrolysis catalyst may be additionally fed along with water before

the hydrolysis reaction. The hydrolysis catalyst may be the same as, or different from, the alcoholysis catalyst.

In addition, upon completion of the hydrolysis reaction, the unreacted components are recovered and reintroduced into the alcoholysis or hydrolysis reaction for reaction one or more times, thereby enhancing the yield of the terephthalic acid finally produced. Further, the amount of waste generated can be reduced, making it environmentally friendly. For example, the filtrate obtained by filtering the excess unreacted alcohol (having 4 or more carbon atoms) and ethylene glycol as a by-product using a filter or the like can be reintroduced into the hydrolysis reaction.

**Process for preparing terephthalic acid**

The process for preparing terephthalic acid according to an embodiment of the present invention comprises (1) subjecting waste polyester to alcoholysis with an alcohol having 4 or more carbon atoms to prepare a liquid composition comprising a compound represented by Formula 1; and (2) subjecting the liquid composition to hydrolysis.

[Formula 1]

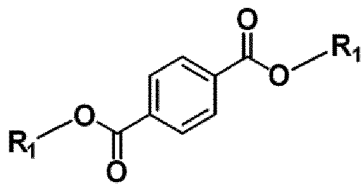

In Formula 1, $R_1$ is an alkyl group having 4 or more carbon atoms.

Alcoholysis step (1)

The process for preparing terephthalic acid according to an embodiment of the present invention comprises subjecting waste polyester to alcoholysis with an alcohol having 4 or more carbon atoms to prepare a liquid composition comprising a compound represented by Formula 1.

The waste polyester may be obtained by crushing or melting waste polyester products. For example, the waste polyester may be obtained by crushing polyester products that have been used up and recovered and separated, or converting them into pellets (post consumer recycled material; PCR), or polyester waste (post industrial recycled material; PIR) such as defective products or scraps that may be formed during processes such as molding of polyester films, fibers, containers, or the like, but it is not limited thereto.

In addition, the number of carbon atoms of the alcohol may be 4 or more, 6 or more, 8 or more, 10 or more, or 12 or more, and may be 4 to 14, 4 to 13, 4 to 10, 4 to 8, 6 to 12, 8 to 14, or 8 to 13.

As the alcoholysis of waste polyester is carried out using an alcohol having the above number of carbon atoms, the alcoholysis can be carried out at a lower temperature and pressure as compared with the conventional process using waste polyester that is carried out at a high temperature and a high pressure, and an intermediate, that is, an alcoholysis product, can be produced in a liquid form. In addition, as the number of carbon atoms of the alcohol satisfies the above range, the alcoholysis reaction rate can be enhanced.

In addition, the alcohol may have a boiling point of 100°C to 290°C. For example, the boiling point of the alcohol may be 100°C to 280°C, 100°C to 260°C, 100°C to 230°C, 110°C to 190°C, or 180°C to 290°C. As the boiling point of the alcohol satisfies the above range, ethylene glycol formed as a by-product in the alcoholysis can be more easily removed or recovered in the subsequent process, thereby further enhancing processability. In particular, there has been a trend in recent years to use various monomer materials in the field in which polyester is used as a raw material; thus, it can be readily adopted for removing various dialcohol-type monomers used in waste polyester products such as waste plastic products.

The weight ratio of the waste polyester to the alcohol may be 1:1 to 10. For example, the weight ratio of the waste polyester to the alcohol may be 1:1 to 8, 1:1 to 6, 1:1 to 4, 1:1 to 3.5, 1: 1.1 to 3.3, 1:2 to 4, or 1:2 to 3.5.

In addition, the alcoholysis reaction may be carried out at a temperature of 160°C to 280°C and a pressure of 1 bar to 40 bar for 0.5 hour to 24 hours. For example, the alcoholysis reaction may be carried out at a temperature of 165°C to 280°C, 165°C to 270°C, 180°C to 270°C, 190°C to 250°C, 200°C to 265°C, 220°C to 265°C, 240°C to 260°C, or 245°C to 260°C and a pressure of 1 bar to 38 bar, 1 bar to 33 bar, 1 bar to 28 bar, 1 bar to 24 bar, 2 bar to 40 bar, 3 bar to 35 bar, or 5 bar to 30 bar for 0.5 hour to 22 hours, 1 hour to 15 hours, 1.5 hours to 10 hours, 2 hours to 8 hours, or 2 hours to 6 hours.

In step (1), an alcoholysis catalyst may be added. Specifically, the alcoholysis reaction can be smoothly carried out as a non-catalytic reaction without using an alcoholysis catalyst, making it environmentally friendly. In particular, when

the content of insoluble metals in waste polyester is high, a non-catalytic reaction may be advantageous for efficient treatment and removal of impurities. In addition, an alcoholysis catalyst may be added in step (1) from the viewpoint of energy to enhance processability by improving reactivity.

The alcoholysis catalyst may be a metal acetate salt, an alkali metal salt, or a hydroxy salt.

More specifically, the alcoholysis catalyst may comprise at least one cation selected from the group consisting of alkali metal ions such as $Li^+$, $Na^+$, $K^+$, and $Cs^+$, alkali earth metal ions such as $Be^{2+}$, $Mg^{2+}$, $Ca^{2+}$, and $Ba^{2+}$, ammonium ions such as $NH^{4+}$ and $NR^{4+}$ (where R is alkyl), and $Zn^{2+}$; or at least one anion selected from the group consisting of $OH^-$, $OR^-$ (where R is alkyl), $HCO_3^-$, $CO_3^{2-}$, benzoate ion ($C_7H_5O_2^-$), 4-alkoxycarbonylbenzoate ion, acetate ion, and terephthalate ion. R may be an alkyl group with 1 to 10 carbon atoms or an alkyl group with 1 to 5 carbon atoms.

For example, the alcoholysis catalyst may comprise at least one selected from the group consisting of $Zn(OAC)_2$, $Co(OAc)_2$, $Mn(OAc)_2$, $Mg(OAc)_2$, $Ca(OAc)_2$, $Ba(OAc)_2$, LiOAc, NaOAc, KOAc, $Zn(OAC)_2 \cdot 2H_2O$, $Co(OAc)_2 \cdot 4H_2O$, $Pb(OAc)_2$, $Mn(OAc)_2 \cdot 4H_2O$, $Mg(OAc)_2 \cdot 4H_2O$, $Pd(OAc)_2$, $Ti(OBu)_4$, $Ti(OiPr)_4$, $GeO_2$, $Al(OiPr)_3$, $Na_2CO_3$, $K_2CO_3$, dibutyltin(IV) oxide, stannous octoate, titanium phosphate, and terephthalic acid.

In addition, the amount of the alcoholysis catalyst added may be 10 ppm to 10,000 ppm based on the total weight of the waste polyester. For example, the amount of the alcoholysis catalyst added may be 10 ppm to 9,000 ppm, 15 ppm to 8,000 ppm, 20 ppm to 6,000 ppm, 50 ppm to 3,500 ppm, 100 ppm to 1,500 ppm, 150 ppm to 1,000 ppm, 180 ppm to 500 ppm, or 200 ppm to 450 ppm, based on the total weight of the waste polyester.

A liquid composition comprising a compound represented by Formula 1 is prepared by the alcoholysis. Specifically, the composition is liquid at room temperature, and the liquid composition is a composition produced through an alcoholysis reaction.

The liquid composition comprises a compound represented by Formula 1.

[Formula 1]

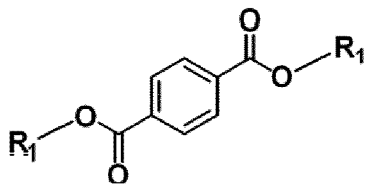

In Formula 1, $R_1$ is an alkyl group having 4 or more carbon atoms.

Specifically, $R_1$ may be butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, hexyl, 1-methylhexyl, 2-ethyl-1-hexyl, heptyl, n-heptyl, 1-methylheptyl, octyl, n-octyl, isooctyl, tert-octyl, 2-propylpentyl, n-nonyl, 2,2-dimethylheptyl, 1-ethyl-propyl, 1,1-dimethylpropyl, isohexyl, 2-methylpentyl, 4-methylhexyl, 5-methylhexyl, decanyl, undecanyl, dodecanyl, tridecanyl, or tetradecanyl.

Specifically, the liquid composition may comprise unreacted alcohol and ethylene glycol (EG) as a by-product. More specifically, the liquid composition may comprise the compound represented by Formula 1, ethylene glycol (EG) formed as a by-product by the alcoholysis reaction, unreacted alcohol, and oligomers.

For example, the liquid composition may comprise a compound represented by Formula 2, and the oligomer may comprise a compound represented by Formula 3.

[Formula 2]

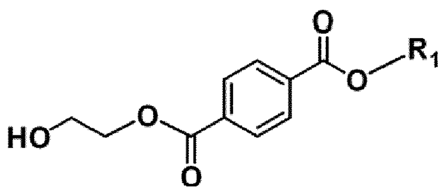

[Formula 3]

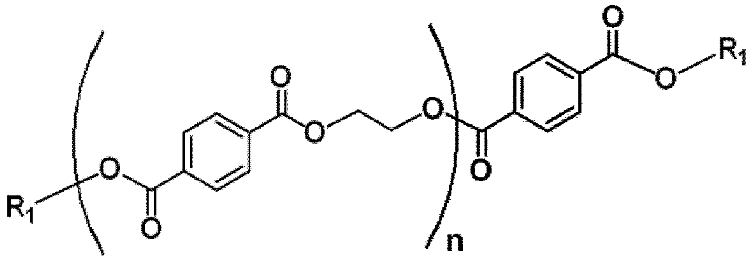

In Formulae 2 and 3, $R_1$ is an alkyl group having 4 or more carbon atoms, and n is an integer of 1 or more.

The liquid composition may comprise unreacted alcohol and ethylene glycol as a by-product, and the content of the compound represented by Formula 1 in the liquid composition may be 70% by mole or more. For example, the content of the compound represented by Formula 1 in the liquid composition prepared by the alcoholysis may be 72% by mole or more, 75% by mole or more, 80% by mole or more, 85% by mole or more, 90% by mole or more, 91% by mole or more, 92% by mole or more, 92.5% by mole or more, 93% by mole or more, 95% by mole or more, 97% by mole or more, 99% by mole or more, or 99.5% by mole or more.

In addition, the content of oligomers in the liquid composition may be 15% by mole or less. For example, the content of oligomers in the liquid composition may be 11% by mole or less, 8.5% by mole or less, 7% by mole or less, 5% by mole or less, 3% by mole or less, 1% by mole or less, 0.8% by mole or less, 0.4% by mole or less, or 0.1% by mole or less.

According to another embodiment of the present invention, step (1) may comprise discharging the unreacted alcohol and ethylene glycol as a by-product.

Specifically, the alcohol is separated from the discharged mixture of alcohol and ethylene glycol, and the separated alcohol may be recycled as a raw material for the alcoholysis. For example, ethylene glycol as a by-product formed in the alcoholysis and unreacted alcohol (alcohol present in excess) are discharged in the form of a gaseous mixture in real-time during the alcoholysis reaction to be subjected to fractional distillation or layer separation and/or subjected to fractional distillation upon completion of the reaction to be separated into alcohol and ethylene glycol. The separated alcohol may be introduced into the alcoholysis in step (1) again and reused. In such an event, the volume and feed rate of the alcohol introduced for reuse may be the same as the volume and discharge rate of the mixture of alcohol and ethylene glycol discharged.

In addition, step (1) may comprise recovering ethylene glycol as a by-product of the alcoholysis. Specifically, ethylene glycol as a by-product of the alcoholysis may be recovered by fractional distillation or layer separation of the mixture of alcohol and ethylene glycol discharged and may be recovered by fractional distillation or layer separation of the composition prepared in step (1).

For example, the unreacted alcohol and ethylene glycol as a by-product are discharged as a gaseous mixture in real-time during the alcoholysis reaction, which is condensed using an external cooling apparatus to recover ethylene glycol.

According to an embodiment of the present invention, not only can unreacted alcohol and ethylene glycol be separated through a simple process such as fractional distillation or layer separation, but the separated unreacted alcohol can also be recycled as a raw material for the alcoholysis, and the recovered ethylene glycol can also be used in other processes. Thus, processability and process cost reduction are excellent.

The recovery rate of ethylene glycol may be 65% or more. For example, the recovery rate of ethylene glycol may be 70% or more, 76% or more, 85% or more, 90% or more, 93% or more, 95% or more, 97% or more, 98% or more, 99% or more, or 99.5% or more.

Purification step

The process for preparing terephthalic acid according to another embodiment of the present invention may further comprise purifying the liquid composition before step (2).

Specifically, the purification step may comprise adding at least one adsorbent selected from the group consisting of activated carbon, silica gel, alumina, zeolite, and activated clay, or adsorption through bed adsorption. More specifically, the adsorbent may be activated carbon or a mixture of activated carbon and silica gel. For example, the adsorbent may be a mixture of activated carbon and silica gel at a weight ratio of 1:0.5 to 1.5 or 1:0.8 to 1.2, but it is not limited thereto.

The content of the adsorbent added may be 0.1% by weight to 20% by weight based on the total weight of the liquid composition. For example, the content of the adsorbent added may be 0.1% by weight to 18% by weight, 0.1% by weight to 15% by weight, 0.1% by weight to 10% by weight, 0.1% by weight to 5% by weight, or 0.1% by weight to 2% by weight, based on the total weight of the liquid composition.

As the step of purifying the liquid composition using the adsorbent, specifically using the adsorbent that satisfies the specific feed amount within the above numerical range, is further carried out, purity and yield can be further enhanced. Specifically, as the step of purifying the liquid composition, which is the alcoholysis reaction composition, is carried out, it is possible to more effectively remove insoluble impurities such as metals that may be contained in the alcoholysis reaction composition, or additives such as colorants and pigments that may be contained in waste polyester. Thus, the purity and yield of the terephthalic acid finally prepared can be further enhanced.

In addition, the process for preparing terephthalic acid according to another embodiment of the present invention may further comprise a concentration step after the purification step.

The concentration may be carried out at a temperature of 50°C to 120°C for 0.5 hour to 6 hours. For example, the concentration may be carried out at a temperature of 55°C to 115°C, 60°C to 110°C, 65°C to 105°C, or 75°C to 100°C for 1 hour to 5 hours, 1.5 hours to 4 hours, or 2 hours to 4 hours by stirring the purified alcoholysis reaction composition.

The purified composition may have a pigment residual rate (%) of 15% or less according to Equation A. For example, the pigment residual rate (%) of the purified alcoholysis reaction composition according to Equation A may be 13% or less, 11% or less, 10% or less, 8% or less, 6% or less, 5.5% or less, 5% or less, 4.3% or less, or 4% or less.

[Equation A]

$$Pigment\ residual\ rate\ of\ liquid\ composition\ (\%) = \frac{A1}{A2} \times 100$$

In Equation A,

A1 is the area of an absorbance curve obtained in 400 nm to 800 nm using a UV-vis spectrophotometer for the purified alcoholysis reaction composition as diluted to a concentration of 5% in dimethyl sulfoxide (DMSO), dimethyl formamide (DMF), or methylpyrrolidone (NMP), and A2 is the area of an absorbance curve obtained in the same manner as above for the alcoholysis reaction composition that has not been purified.

The description on purification in Equation A is as described above.

The pigment residual rate refers to the content of additives such as colorants, pigments, dyes, or the like remaining in the composition. The lower the pigment residual rate, the lower the content of additives such as colorants, pigments, dyes, or the like, indicating higher purity.

In addition, the purified composition may have a low content of insoluble impurities such as metals. Specifically, the purified liquid composition may have a total metal content of 100 ppm or less relative to the total weight of the purified alcoholysis reaction composition when measured by inductively coupled plasma atomic emission spectroscopy (ICP-AES).

For example, the purified alcoholysis reaction composition may contain insoluble impurities such as metals. The total content of metals in the purified alcoholysis reaction composition may be 80 ppm or less, 70 ppm or less, 60 ppm or less, 50 ppm or less, 40 ppm or less, or less than 30 ppm, relative to the total weight of the purified alcoholysis reaction composition, when measured by inductively coupled plasma atomic emission spectroscopy (ICP-AES). In particular, the total content of Sb, Ti, and Zn in the purified alcoholysis reaction composition may be less than 30 ppm, 25 ppm or less, 20 ppm or less, 15 ppm or less, 10 ppm or less, 5 ppm or less, 3 ppm or less, or 1 ppm or less.

Sb is known to be a widely used catalyst in the polymerization of common polyester by virtue of its excellent stability, reaction rate, and cost. As regulations are being strengthened due to the impact of Sb on the human body and the environment, however, it is a substance that must be removed during the chemical recycling process.

In addition, Ti may be used as a catalyst for polyester polymerization or as an additive in polyester processing in the form of $TiO_2$. If it is contained in a certain amount or more, the quality of recycled terephthalic acid prepared therefrom or a polyester resin using it may deteriorate, thereby limiting its use.

Zn is also a component used as a polymerization catalyst for polyester such as PET. If it remains, it may affect the control of reactivity in the preparation process of recycled terephthalic acid or a polyester resin using it. Thus, it is preferable to be removed. In particular, since it is widely used in the chemical recycling process, it is a substance that requires sufficient removal of the amount contained in waste plastic, which is the raw material for the present process, as well as the amount separately added as a catalyst during the recycling process.

According to an embodiment of the present invention, as the purification is further carried out, the total content of metals, especially Sb, Ti, and Zn as described above, in the purified alcoholysis reaction composition is very low at 30 ppm or less.

For example, the content of Sb may be 30 ppm or less, 20 ppm or less, 10 ppm or less, or 1 ppm or less, relative to the total weight of the purified alcoholysis reaction composition, when measured by inductively coupled plasma atomic emission spectroscopy (ICP-AES).

The content of Ti may be 30 ppm or less, 20 ppm or less, 10 ppm or less, or 1 ppm or less, relative to the total weight of the purified alcoholysis reaction composition, when measured by inductively coupled plasma atomic emission

spectroscopy (ICP-AES).

The content of Zn may be 30 ppm or less, 20 ppm or less, 10 ppm or less, or 1 ppm or less, relative to the total weight of the purified alcoholysis reaction composition, when measured by inductively coupled plasma atomic emission spectroscopy (ICP-AES).

Hydrolysis reaction step (2)

The process for preparing terephthalic acid according to an embodiment of the present invention comprises subjecting the composition to hydrolysis. Specifically, in step (2), the alcoholysis reaction composition (liquid composition comprising the compound represented by Formula 1) prepared in step (1), or the purified alcoholysis reaction composition, may be subjected to hydrolysis to prepare recycled terephthalic acid.

The hydrolysis may be carried out by adding water to the composition. For example, the hydrolysis may be carried out by adding water to the alcoholysis reaction composition, or the purified alcoholysis reaction composition, at a temperature of 180°C to 280°C, 185°C to 280°C, 200°C to 275°C, 220°C to 270°C, or 240°C to 265°C, for 0.5 hour to 24 hours, 1 hour to 20 hours, 2.5 hours to 12 hours, or 3 hours to 8 hours.

The conventional method in which a metal catalyst such as iron, cobalt, manganese, or nickel is added to waste polyester, which is then directly hydrolyzed with water, is environmentally friendly. However, processability is low in that very high-temperature conditions of 300°C or higher are required, and the reaction apparatus must also have high-pressure resistance. In contrast, the process for preparing terephthalic acid according to an embodiment of the present invention has excellent processability since the process conditions are ameliorated as compared with the prior art.

In addition, the weight ratio of the composition to water may be 1:1 to 500. For example, the weight ratio of the alcoholysis reaction composition (purified, or purified and concentrated, alcoholysis reaction composition) used in the hydrolysis to water may be 1:1 to 450, 1:1 to 400, 1:1 to 250, 1:1 to 100, 1:1 to 50, 1:1.2 to 20, or 1:1.5 to 10.

In addition, in step (2), a hydrolysis catalyst may be added. Specifically, a hydrolysis catalyst may be added to the mixture of the alcoholysis reaction composition and water to carry out hydrolysis.

The hydrolysis reaction can be smoothly carried out as a non-catalytic reaction without using a hydrolysis catalyst, making it environmentally friendly. In particular, when the content of insoluble metals in waste polyester is high, a non-catalytic reaction may be advantageous for efficient treatment and removal of impurities. In addition, a hydrolysis catalyst may be added in step (2) from the viewpoint of energy to enhance processability by improving reactivity.

The hydrolysis catalyst may be a metal acetate salt, an alkali metal salt, or a hydroxy salt.

More specifically, the hydrolysis catalyst may comprise at least one cation selected from the group consisting of alkali metal ions such as $Li^+$, $Na^+$, $K^+$, and $Cs^+$, alkali earth metal ions such as $Be^{2+}$, $Mg^{2+}$, $Ca^{2+}$, and $Ba^{2+}$, ammonium ions such as $NH^{4+}$ and $NR^{4+}$ (where R is alkyl), and $Zn^{2+}$; or at least one anion selected from the group consisting of $OH^-$, $OR^-$ (where R is alkyl), $HCO_3^-$, $CO_3^{2-}$, benzoate ion ($C_7H_5O_2^-$), 4-alkoxycarbonylbenzoate ion, acetate ion, and terephthalate ion. R may be an alkyl group with 1 to 10 carbon atoms or an alkyl group with 1 to 5 carbon atoms.

For example, the hydrolysis catalyst may comprise at least one selected from the group consisting of $Zn(OAC)_2$, $Co(OAc)_2$, $Mn(OAc)_2$, $Mg(OAc)_2$, $Ca(OAc)_2$, $Ba(OAc)_2$, LiOAc, NaOAc, KOAc, $Zn(OAC)_2 \cdot 2H_2O$, $Co(OAc)_2 \cdot 4H_2O$, $Pb(OAc)_2$, $Mn(OAc)_2 \cdot 4H_2O$, $Mg(OAc)_2 \cdot 4H_2O$, $Pd(OAc)_2$, $Ti(OBu)_4$, $Ti(OiPr)_4$, $GeO_2$, $Al(OiPr)_3$, $Na_2CO_3$, $K_2CO_3$, dibutyltin(IV) oxide, stannous octoate, titanium phosphate, and terephthalic acid.

In addition, the amount of the hydrolysis catalyst added may be 10 ppm to 10,000 ppm based on the total weight of the composition. For example, the amount of the hydrolysis catalyst added may be 15 ppm to 8,000 ppm, 20 ppm to 5,500 ppm, 30 ppm to 3,000 ppm, 50 ppm to 1,600 ppm, 100 ppm to 1,200 ppm, 150 ppm to 1,100 ppm, 300 ppm to 1,000 ppm, 350 ppm to 950 ppm, 400 ppm to 850 ppm, 420 ppm to 700 ppm, or 450 ppm to 650 ppm, based on the total weight of the alcoholysis reaction composition (purified, or purified and concentrated, alcoholysis reaction composition).

According to an embodiment of the present invention, solid terephthalic acid may be prepared through the hydrolysis reaction. Specifically, the process may further comprise, after the hydrolysis step, filtering, washing, and drying the hydrolysis reaction product prepared by the hydrolysis reaction. That is, the hydrolysis reaction product prepared through the hydrolysis reaction may be filtered, washed, and dried to produce solid terephthalic acid.

For example, the hydrolysis reaction product may be cooled to an appropriate temperature, for example, from room temperature to less than 100°C, at which water does not vaporize, to obtain a solution in the form of a slurry, which is filtered to obtain a solid, which is washed and dried under vacuum to obtain solid terephthalic acid.

The washing may be carried out using a mixture of an alcohol having 4 or more carbon atoms and/or water, a protic solvent such as isopropanol and acetic acid, or an aprotic solvent such as acetone, dichloromethane, chloroform, tetrahydrofuran (THF), and toluene.

Here, the description on the alcohol having 4 or more carbon atoms is as described above. The alcohol with 4 or more carbon atoms used in the washing may be the same as, or different from, the alcohol with 4 or more carbon atoms used in step (1). For example, when 1-butanol is used as alcohol in step (1) for producing terephthalic acid, the solid terephthalic acid prepared may be washed with a mixture of 1-butanol and water. In addition, the number of carbon atoms

in the alcohol used in the washing may be the same as the number of carbon atoms in the alcohol used in step (1).

Residual pigments or impurities generated by the decomposition of pigments during hydrolysis, especially yellow impurities, can be effectively removed through the washing, thereby enhancing yellow index or color characteristics. In addition, as water is used for washing, mineral salts can be removed, thereby enhancing quality.

In addition, the yield of terephthalic acid may be 65% or more. For example, the yield of the recycled terephthalic acid finally produced may be 68% or more, 70% or more, 75% or more, 80% or more, 85% or more, 90% or more, or 95% or more.

Recycled terephthalic acid

Recycled terephthalic acid is prepared according to the above process for preparing terephthalic acid and has a total metal content of less than 100 ppm when measured by inductively coupled plasma atomic emission spectroscopy (ICP-AES).

Specifically, the recycled terephthalic acid may be prepared according to the process for preparing terephthalic acid.

The recycled terephthalic acid may have a total metal content of less than 100 ppm, 90 ppm or less, 80 ppm or less, 65 ppm or less, 50 ppm or less, 35 ppm or less, less than 30 ppm, 15 ppm or less, 9 ppm or less, 7 ppm or less, 5 ppm or less, or 1 ppm or less, when measured by inductively coupled plasma atomic emission spectroscopy (ICP-AES).

In addition, the recycled terephthalic acid may have a total content of Sb, Ti, and Zn of less than 30 ppm when measured by inductively coupled plasma atomic emission spectroscopy (ICP-AES). For example, the total content of Sb, Ti, and Zn, which may be harmful to the human body or have the potential to serve as a catalyst for reaction or side reaction in the subsequent polymerization process, in the recycled terephthalic acid may be 25 ppm or less, 20 ppm or less, 15 ppm or less, 10 ppm or less, 5 ppm or less, 3 ppm or less, or 1 ppm or less.

For example, the content of Sb in the recycled terephthalic acid may be 30 ppm or less, 25 ppm or less, 20 ppm or less, 15 ppm or less, 10 ppm or less, 5 ppm or less, 3 ppm or less, or 1 ppm or less, relative to the total weight of the recycled terephthalic acid, when measured by inductively coupled plasma atomic emission spectroscopy (ICP-AES).

The content of Ti in the recycled terephthalic acid may be 30 ppm or less, 25 ppm or less, 20 ppm or less, 15 ppm or less, 10 ppm or less, 5 ppm or less, 3 ppm or less, or 1 ppm or less, relative to the total weight of the recycled terephthalic acid, when measured by inductively coupled plasma atomic emission spectroscopy (ICP-AES).

The content of Zn in the recycled terephthalic acid may be 30 ppm or less, 25 ppm or less, 20 ppm or less, 15 ppm or less, 10 ppm or less, 5 ppm or less, 3 ppm or less, or 1 ppm or less, relative to the total weight of the recycled terephthalic acid, when measured by inductively coupled plasma atomic emission spectroscopy (ICP-AES).

The recycled terephthalic acid may have a color-b of less than 2, 1.6 or less, 1.4 or less, 1.3 or less, or 1 or less, as measured with a colorimeter. Since the above numerical range of color-b is equivalent to that of virgin terephthalic acid commonly produced in the petrochemical process, the recycled terephthalic acid that satisfies the above range of color-b is not only low in yellow index but also excellent in quality as the monomer is well purified.

Col-b is a color coordinate established by the Commission International d'Eclairage (CIE), where color is represented by L (brightness), a (green to red complementary color), and b (yellow to blue complementary color). It can be measured using a colorimeter.

In addition, the recycled terephthalic acid may have a pigment residual rate of 10% or less according to Equation B. For example, the pigment residual rate of the recycled terephthalic acid may be 10% or less, 8% or less, 7% or less, 5% or less, or 4% or less.

[Equation B]

$$Pigment\ residual\ rate\ of\ recycled\ terephthalic\ acid\ (\%) = \frac{B1}{B2} \times 100$$

In Equation B,
B1 is the area of an absorbance curve obtained in 400 nm to 800 nm using a UV-vis spectrophotometer for the recycled terephthalic acid as diluted to a concentration of 5% in dimethyl sulfoxide (DMSO), dimethyl formamide (DMF), or methylpyrrolidone (NMP), and B2 is the area of an absorbance curve obtained in the same manner as above for the recycled terephthalic acid that has not been purified in the preparation process thereof.

B1 may be measured for recycled terephthalic acid produced by performing a purification step, or a purification and concentration step, in the process for preparing recycled terephthalic acid. B2 may be measured for recycled terephthalic acid produced by without performing a purification step, or performing a concentration step alone, in the process for preparing recycled terephthalic acid.

In addition, the yellow index (Y.I.) may be less than 2, 1.8 or less, or 1.7 or less when the recycled terephthalic acid is diluted to a concentration of 5% in dimethyl sulfoxide (DMSO), dimethyl formamide (DMF), and methylpyrrolidone (NMP), respectively, and measured. The yellow index may be measured for recycled terephthalic acid produced by performing a purification step, or a purification and concentration step, in the process for preparing recycled terephthalic acid.

## Polyester resin and process for preparing the same

Also disclosed herein is a polyester resin that comprises the recycled terephthalic acid.

Specifically, the polyester resin may comprise the recycled terephthalic acid, a diol compound or a derivative thereof, and optionally a dicarboxylic acid compound or a derivative thereof.

For example, the diol component or a derivative thereof may comprise at least one selected from the group consisting of ethylene glycol, monoethylene glycol, diethylene glycol, 1,4-butanediol, 1,3-propanediol, 1,4-cyclohexanedimethanol, and neopentyl glycol. The dicarboxylic acid or a derivative thereof may comprise at least one selected from the group consisting of terephthalic acid (TPA), isophthalic acid (IPA), 2,6-naphthalenedicarboxylic acid (2,6-NDA), dimethyl terephthalate (DMT), dimethylisophthalate (DMI), and dimethyl 2,6-naphthalenedicarboxylate (2,6-NDC). But they are not limited thereto.

The process for preparing a polyester resin according to another embodiment of the present invention comprises mixing the recycled terephthalic acid with a diol compound or a derivative thereof, and optionally a dicarboxylic acid compound or a derivative thereof, and then carrying out an esterification reaction; and subjecting the esterification reaction product to a polycondensation reaction.

The esterification reaction may be carried out at a temperature of 200°C to 350°C, 220°C to 320°C, or 250°C to 290°C. In addition, the esterification reaction may be carried out in a pressurized state higher than normal pressure by 0 $kg/cm^2$ to 10 $kg/cm^2$ (0 mmHg to 7,355.6 mmHg), 0 $kg/cm^2$ to 5 $kg/cm^2$ (0 to 3,677.8 mmHg) or 0 $kg/cm^2$ to 2.0 $kg/cm^2$ (0 to 1,471.1 mmHg). In addition, the esterification reaction may be carried out for 1 hour to 24 hours, 1 hour to 10 hours, or 1 hour to 6 hours.

In addition, the polycondensation reaction may be carried out at a temperature of 150°C to 400°C, 200°C to 370°C, 250°C to 350°C, or 270°C to 300°C. In addition, the polycondensation reaction may be carried out at a reduced pressure of 0.01 mmHg to 400 mmHg, 0.05 mmHg to 100 mmHg, or 0.1 mmHg to 100 mmHg. In addition, the polycondensation reaction may be carried out for the required time until the desired intrinsic viscosity is reached. For example, it may be carried out for 1 hour to 24 hours, 1 hour to 10 hours, or 1 hour to 4 hours.

In addition, a catalyst and/or a stabilizer may be further added in the esterification reaction and the polycondensation reaction.

For example, the catalyst for an esterification reaction may be methylates of sodium and magnesium; acetates, borates, fatty acid salts, and carbonates of Zn, Cd, Mn, Co, Ca, and Ba; metallic Mg; and oxides of Pb, Zn, Sb, and Ge.

In addition, the catalyst for polycondensation reaction may be, for example, titanium-based catalysts such as tetraethyl titanate, acetyl tripropyl titanate, tetrapropyl titanate, tetrabutyl titanate, polybutyl titanate, 2-ethylhexyl titanate, octylene glycol titanate, lactate titanate, triethanolamine titanate, acetyl acetonate titanate, ethyl acetoacetic ester titanate, isostearyl titanate, titanium dioxide, titanium dioxide/silicon dioxide copolymers, titanium dioxide/zirconium dioxide copolymers; germanium-based catalysts such as germanium dioxide and copolymers using the same; or tin-based catalysts such as monobutyltin oxide, dibutyltinoxide, and monobutylhydroxytinoxide.

In addition, the stabilizer may be a phosphorus-based compound such as phosphoric acid, trimethyl phosphate, and triethyl phosphate may be used, but it is not limited thereto.

The process for preparing a polyester resin according to another embodiment of the present invention may further comprises carrying out a solid-state polymerization reaction. For example, the solid-state polymerization may be carried out after the polycondensation reaction at a temperature of 190°C to 230°C under vacuum conditions of 0.2 Torr to 2.0 Torr or a nitrogen atmosphere.

Mode for the Invention

Hereinafter, the present invention will be described in more detail with reference to the following examples. However, these examples are set forth to illustrate the present invention, and the scope of the present invention is not limited thereto.

[Example]

Preparation of a liquid composition

Example 1-1

A first high-pressure reactor with a capacity of 7 liters was charged with 1 kg of waste polyethylene terephthalate (waste PET) and 3.3 kg of 1-butanol as an alcohol, followed by an addition thereto of 200 mg of $Zn(OAC)_2 \cdot 2H_2O$ as an alcoholysis catalyst (200 ppm relative to the total weight of the waste PET).

Thereafter, with all connections of the first high-pressure reactor tightened and sealed, the temperature was raised to 250°C over 1 hour, and an alcoholysis reaction was carried out with stirring for 3 hours while a temperature of 250°C and a pressure of 24 bar were maintained.

Upon completion of the alcoholysis reaction, it was cooled to room temperature to obtain a liquid alcoholysis reaction composition. Here, the components and contents of the alcoholysis reaction composition were analyzed by NMR. The alcoholysis reaction composition contained a compound represented by Formula 1 ($R_1$: $(CH_2)_3CH_3$), residual ethylene glycol (EG), an alcohol (1-butanol), and oligomers.

Thereafter, the alcoholysis reaction composition was charged to a separate flask, and excess unreacted 1-butanol and produced ethylene glycol (EG) were recovered using a fractional distillation apparatus, respectively.

[Formula 1]

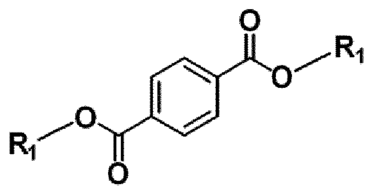

Example 1-2

A first high-pressure reactor with a capacity of 7 liters was charged with 1 kg of waste polyethylene terephthalate (waste PET) and 3.3 kg of 1-butanol as an alcohol, followed by an addition thereto of 200 mg of $Zn(OAC)_2 \cdot 2H_2O$ as an alcoholysis catalyst (200 ppm relative to the total weight of the waste PET).

Thereafter, with all connections of the first high-pressure reactor tightened and sealed, the temperature was raised to 250°C over 1 hour, and an alcoholysis reaction was carried out with stirring for 3 hours while a temperature of 250°C and a pressure of 24 bar were maintained.

Specifically, one hour after the alcoholysis reaction began, the valve of the back pressure regulator installed in advance was adjusted to discharge a gaseous mixture of ethylene glycol (EG), a by-product produced by the alcoholysis reaction, and 1-butanol present in excess. In such an event, the internal temperature of the first high-pressure reactor was maintained at 250°C, and the gaseous mixture of ethylene glycol (EG) and 1-butanol discharged through the back pressure regulator was condensed using an external cooling device. In addition, the discharge rate of the gaseous mixture of ethylene glycol (EG) and 1-butanol was adjusted to 3 kg/h, and, at the same time, 1-butanol was continuously supplied to the first high-pressure reactor. In such an event, the volume and feed rate of 1-butanol newly supplied to the first high-pressure reactor were adjusted to be the same as the volume and discharge rate of the gaseous mixture of ethylene glycol (EG) and 1-butanol discharged. An alcoholysis reaction was carried out while the discharge and feed processes were maintained for 3 hours.

Upon completion of the alcoholysis reaction, it was cooled to room temperature to obtain a liquid alcoholysis reaction composition. Here, the components and contents of the alcoholysis reaction composition were analyzed by NMR. The alcoholysis reaction composition contained a compound represented by Formula 1 ($R_1$: $(CH_2)_3CH_3$), residual ethylene glycol (EG), an alcohol (1-butanol), and oligomers.

Thereafter, the alcoholysis reaction composition was charged to a separate flask, and excess unreacted 1-butanol and produced ethylene glycol were recovered using a fractional distillation apparatus, respectively.

[Formula 1]

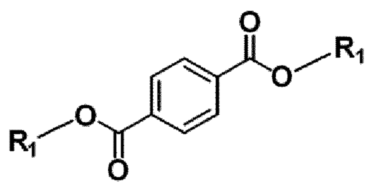

**Example 1-3**

A first high-pressure reactor with a capacity of 7 liters was charged with 1 kg of waste polyethylene terephthalate (waste PET) and 3.3 kg of 1-butanol as an alcohol, followed by an addition thereto of 200 mg of $Zn(OAC)_2 \cdot 2H_2O$ as an alcoholysis catalyst (200 ppm relative to the total weight of the waste PET).

Thereafter, with all connections of the first high-pressure reactor tightened and sealed, the temperature was raised to 250°C over 1 hour, and an alcoholysis reaction was carried out with stirring for 3 hours while a temperature of 250°C and a pressure of 24 bar were maintained.

Specifically, one hour after the alcoholysis reaction began, the valve of the back pressure regulator installed in advance was adjusted to discharge a gaseous mixture of ethylene glycol (EG), a by-product produced by the alcoholysis reaction, and 1-butanol present in excess. In such an event, the internal temperature of the first high-pressure reactor was maintained at 250°C, and the gaseous mixture of ethylene glycol (EG) and 1-butanol discharged through the back pressure regulator was condensed using an external cooling device. In addition, the discharge rate of the gaseous mixture of ethylene glycol (EG) and 1-butanol was adjusted to 3 kg/h, and, at the same time, 1-butanol was continuously supplied to the first high-pressure reactor. In such an event, the volume and feed rate of 1-butanol newly supplied to the first high-pressure reactor were adjusted to be the same as the volume and discharge rate of the gaseous mixture of ethylene glycol (EG) and 1-butanol discharged.

The condensed gaseous mixture of ethylene glycol (EG) and 1-butanol was transferred to a layer separation apparatus with a capacity of 5 liters filled with 3 kg of water, and a procedure of stirring for 10 minutes and layer separation for 2 minutes was repeated to separate a 1-butanol layer and a water and ethylene glycol (EG) layer. The separated 1-butanol was fed to the first high-pressure reactor again in real-time using a high-pressure pump. An alcoholysis reaction was carried out while the procedure of stirring, layer separation, and reintroduction was maintained for 3 hours.

Upon completion of the alcoholysis reaction, it was cooled to room temperature to obtain a liquid alcoholysis reaction composition. Here, the components and contents of the alcoholysis reaction composition were analyzed by NMR. The alcoholysis reaction composition contained a compound represented by Formula 1 ($R_1$: $(CH_2)_3CH_3$), residual ethylene glycol (EG), an alcohol (1-butanol), and oligomers.

Thereafter, the alcoholysis reaction composition was charged to a separate flask, and excess unreacted 1-butanol and produced ethylene glycol were recovered using a fractional distillation apparatus, respectively.

[Formula 1]

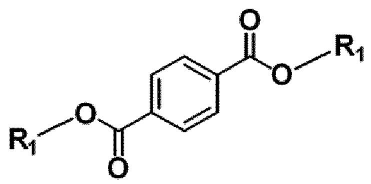

**Example 1-4**

A liquid alcoholysis reaction composition was prepared in the same manner as in Example 1-1, except that 3.3 kg of 1-pentanol was used as an alcohol, and the pressure was maintained at 13 bar.

**Example 1-5**

A liquid alcoholysis reaction composition was prepared in the same manner as in Example 1-2, except that 3.3 kg of 1-pentanol was used as an alcohol, and the pressure was maintained at 13 bar.

**Example 1-6**

A liquid alcoholysis reaction composition was prepared in the same manner as in Example 1-3, except that 3.3 kg of 1-pentanol was used as an alcohol, and the pressure was maintained at 13 bar.

**Example 1-7**

A liquid alcoholysis reaction composition was prepared in the same manner as in Example 1-1, except that 3.3 kg of 1-octanol was used as an alcohol, and the pressure was maintained at 3.4 bar.

**Example 1-8**

A liquid alcoholysis reaction composition was prepared in the same manner as in Example 1-2, except that 3.3 kg of 1-octanol was used as an alcohol, and the pressure was maintained at 3.4 bar.

**Example 1-9**

A liquid alcoholysis reaction composition was prepared in the same manner as in Example 1-3, except that 3.3 kg of 1-octanol was used as an alcohol, and the pressure was maintained at 3.4 bar.

**Example 1-10**

A liquid alcoholysis reaction composition was prepared in the same manner as in Example 1-1, except that 3.3 kg of 2-ethyl-1-hexanol was used as an alcohol, and the pressure was maintained at 4.1 bar.

**Example 1-11**

A liquid alcoholysis reaction composition was prepared in the same manner as in Example 1-2, except that 3.3 kg of 2-ethyl-1-hexanol was used as an alcohol, and the pressure was maintained at 4.1 bar.

**Example 1-12**

A liquid alcoholysis reaction composition was prepared in the same manner as in Example 1-3, except that 3.3 kg of 2-ethyl-1-hexanol was used as an alcohol, and the pressure was maintained at 4.1 bar.

**Example 1-13**

A liquid alcoholysis reaction composition was prepared in the same manner as in Example 1-1, except that 3.3 kg of 1-decanol was used as an alcohol, and the pressure was maintained at 1.6 bar.

**Example 1-14**

A liquid alcoholysis reaction composition was prepared in the same manner as in Example 1-2, except that 3.3 kg of 1-decanol was used as an alcohol, and the pressure was maintained at 1.6 bar.

Example **1-15**

A liquid alcoholysis reaction composition was prepared in the same manner as in Example 1-3, except that 3.3 kg of 1-decanol was used as an alcohol, and the pressure was maintained at 1.6 bar.

**Example 1-16**

A liquid alcoholysis reaction composition was prepared in the same manner as in Example 1-1, except that 3.3 kg of 1-dodecanol was used as an alcohol, and the pressure was maintained at 1.0 bar.

**Example 1-17**

A first high-pressure reactor with a capacity of 7 liters was charged with 1 kg of waste polyethylene terephthalate (waste PET) and 3.3 kg of 1-dodecanol as an alcohol, followed by an addition thereto of 200 mg of $Zn(OAC)_2 \cdot 2H_2O$ as an alcoholysis catalyst (200 ppm relative to the total weight of the waste PET).

Thereafter, with all connections of the first high-pressure reactor tightened and sealed, the temperature was raised to 250°C over 1 hour, and an alcoholysis reaction was carried out with stirring for 3 hours while a temperature of 250°C and a pressure of 1.0 bar were maintained.

Specifically, one hour after the alcoholysis reaction began, the valve of the back pressure regulator installed in advance was adjusted to discharge the vapor of ethylene glycol (EG), a by-product produced by the alcoholysis reaction. In such an event, the internal temperature of the first high-pressure reactor was maintained at 250°C, and the vapor of ethylene glycol (EG) was condensed using an external cooling device.

In addition, 1-dodecanol was continuously supplied to the first high-pressure reactor in an amount equal to the

amount of ethylene glycol (EG) discharged as vapor and condensed. In such an event, the volume and feed rate of 1-dodecanol newly supplied to the first high-pressure reactor were adjusted to be the same as the volume and discharge rate of the vapor of ethylene glycol (EG) discharged. An alcoholysis reaction was carried out while the discharge and feed processes were maintained for 3 hours.

Upon completion of the alcoholysis reaction, it was cooled to room temperature to obtain a liquid alcoholysis reaction composition. Here, the components and contents of the alcoholysis reaction composition were analyzed by NMR. The alcoholysis reaction composition contained a compound represented by Formula 1 ($R_1$: $(CH_2)_{11}CH_3$), residual ethylene glycol (EG), an alcohol (1-dodecanol), and oligomers.

Thereafter, the alcoholysis reaction composition was charged to a separate flask, and excess unreacted 1-dodecanol and produced ethylene glycol (EG) were recovered using a fractional distillation apparatus, respectively.

[Formula 1]

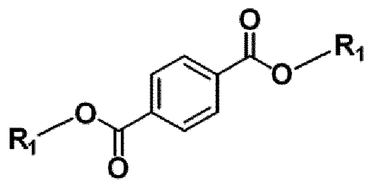

**Example 1-18**

A liquid alcoholysis reaction composition was prepared in the same manner as in Example 1-1, except that 3.3 kg of 1-tetradecanol was used as an alcohol, and the pressure was maintained at 1.0 bar.

**Example 1-19**

A liquid alcoholysis reaction composition was prepared in the same manner as in Example 1-17, except that 3.3 kg of 1-tetradecanol was used as an alcohol.

**Comparative Example 1-1**

A liquid alcoholysis reaction composition was prepared in the same manner as in Example 1-1, except that 3.3 kg of methanol was used as an alcohol, and the pressure was maintained at 83 bar.

**Comparative Example 1-2**

A liquid alcoholysis reaction composition was prepared in the same manner as in Example 1-2, except that 3.3 kg of methanol was used as an alcohol, and the pressure was maintained at 83 bar.

**Comparative Example 1-3**

A liquid alcoholysis reaction composition was prepared in the same manner as in Example 1-1, except that 3.3 kg of ethanol was used as an alcohol, and the pressure was maintained at 64 bar.

**Comparative Example 1-4**

A liquid alcoholysis reaction composition was prepared in the same manner as in Example 1-2, except that 3.3 kg of ethanol was used as an alcohol, and the pressure was maintained at 64 bar.

[Table 1]

| | Alcohol | Liquid composition | | | | Recovery rate of EG (%) | Process pressure (bar) |
|---|---|---|---|---|---|---|---|
| | | Compound of Formula 1 | | EG and alcohol (% by mole) | Oligomer (% by mole) | | |
| | | $R_1$ | content (% by mole) | | | | |
| Ex. 1-1 | 1-butanol | $-(CH_2)_3CH_3$ | 91.3 | 8.1 | 0.6 | 94.0 | 24 |
| Ex. 1-2 | | | 93.2 | 6.2 | 0.6 | 95.1 | 24 |
| Ex. 1-3 | | | 92.7 | 6.6 | 0.7 | 96.2 | 24 |
| Ex. 1-4 | 1-pentanol | $-(CH_2)_4CH_3$ | 92.1 | 7.4 | 0.5 | 97.5 | 13 |
| Ex. 1-5 | | | 97.3 | 2.4 | 0.3 | 93.3 | 13 |
| Ex. 1-6 | | | 97.8 | 2.0 | 0.2 | 98.1 | 13 |
| Ex. 1-7 | 1-octanol | $-(CH_2)_7CH_3$ | 93.3 | 6.1 | 0.6 | 92.1 | 3.4 |
| Ex. 1-8 | | | 99.5 | 0.4 | <0.1 | 99.6 | 3.4 |
| Ex. 1-9 | | | 99.6 | 0.3 | <0.1 | 99.7 | 3.4 |
| Ex. 1-10 | 2-ethyl-1-hexanol | | 93.6 | 5.0 | 1.4 | 92.3 | 4.1 |
| Ex. 1-11 | | | 99.7 | 0.2 | <0.1 | 99.8 | 4.1 |
| Ex. 1-12 | | | 99.7 | 0.2 | <0.1 | 99.6 | 4.1 |
| Ex. 1-13 | 1-decanol | $-(CH_2)_9CH_3$ | 91.9 | 6.5 | 1.6 | 91.8 | 1.6 |
| Ex. 1-14 | | | 99.5 | 0.4 | <0.1 | 99.6 | 1.6 |
| Ex. 1-15 | | | 99.6 | 0.3 | <0.1 | 99.5 | 1.6 |
| Ex. 1-16 | 1-dodecanol | $-(CH_2)_{11}CH_3$ | 91.0 | 8.3 | 0.7 | 92.2 | 1.0 |
| Ex. 1-17 | | | 99.6 | 0.3 | <0.1 | 99.7 | 1.0 |
| Ex. 1-18 | 1-tetradecanol | $-(CH_2)_{13}CH_3$ | 90.0 | 8.1 | 1.9 | 93.3 | 1.0 |
| Ex. 1-19 | | | 99.6 | 0.3 | <0.1 | 99.6 | 1.0 |
| C. Ex. 1-1 | methanol | $-CH_3$ | 82.2 | 13.1 | 2.7 | 85.5 | 83 |
| C. Ex. 1-2 | methanol | $-CH_3$ | 85.1 | 12.3 | 2.6 | 84.2 | 83 |
| C. Ex. 1-3 | ethanol | $-CH_2CH_3$ | 77.5 | 13.0 | 9.5 | 80.0 | 64 |
| C. Ex. 1-4 | ethanol | $-CH_2CH_3$ | 80.2 | 15.5 | 4.3 | 78.3 | 64 |

As can be seen from Table 1 above, as the liquid alcoholysis reaction compositions of Examples 1-1 to 1-19 were prepared using an alcohol having 4 or more carbon atoms, they could be prepared at a very low process pressure as compared with Comparative Examples 1-1 to 1-4. The content of the compound represented by Formula 1, that is, the yield and purity, was high, and the recovery rate of ethylene glycol was also excellent.

**Purification and concentration of the liquid composition**

**Example 2-1**

0.1 g of activated carbon as an adsorbent was added to 100 g of the liquid alcoholysis reaction composition before recovering the excess unreacted alcohol and produced ethylene glycol using a fractional distillation apparatus in Example 1-1, followed by purification thereof, which was stirred at 100°C for 3 hours and then filtered to concentrate the alcoholysis reaction composition.

**Examples 2-2 to 2-23, and Comparative Examples 2-1 and 2-2**

The liquid alcoholysis reaction compositions were each purified and concentrated in the same manner as in

Example 2-1, except that the liquid composition and process conditions were changed as shown in Table 2 below. Here, in Comparative Examples 2-1 and 2-2, it was precipitated in an undissolved state, which was not purified.

**Examples 2-24 to 2-26**

0.1 g of activated carbon as an adsorbent was added to 100 g of the liquid alcoholysis reaction compositions after recovering the excess unreacted alcohol and produced ethylene glycol using a fractional distillation device in each of Examples 1-1, 1-4, and 1-10, followed by purification thereof, which was stirred at 100°C for 3 hours and then filtered to concentrate the alcoholysis reaction composition.

**Comparative Examples 2-3 to 2-21**

An adsorbent was not added to the liquid alcoholysis reaction composition before recovering the excess unreacted alcohol and produced ethylene glycol using a fractional distillation device in each of Examples 1-1 to 1-19, which was stirred at 100°C for 3 hours and then filtered to concentrate it.

**Test Example 1-1: Pigment residual rate**

The compositions of Examples 2-1 to 2-26 and Comparative Examples 2-1 to 2-21 were each measured for pigment residual rate (%).

Specifically, the composition of Example 2-1 was diluted to a concentration of 5% in dimethyl sulfoxide (DMSO), dimethyl formamide (DMF), and methylpyrrolidone (NMP), respectively, and the area (A1) of an absorbance curve was obtained in 400 nm to 800 nm using a UV-vis spectrophotometer. In addition, for the composition of Comparative Example 2-3 (the composition of Example 1-1 was used in the same manner as the composition of Example 2-1 above whereas the composition was concentrated without purification by adding an adsorbent), the area (A2) of an absorbance curve was obtained in 400 nm to 800 nm in the same manner as above. The pigment residual rate (%) was calculated according to the following Equation A using the area of the measured absorbance curve.

[Equation A]

$$Pigment\ residual\ rate\ of\ liquid\ composition\ (\%) = \frac{A1}{A2} \times 100$$

The compositions of Examples 2-2 to 2-26 and Comparative Examples 2-1 to 2-21 were each measured for pigment residual rate (%) in the same manner as above.

**Test Example 1-2: Yellow index**

The compositions of Examples 2-1 to 2-26 and Comparative Examples 2-1 to 2-21 were each measured for yellow index (Y.I.).

Specifically, the compositions of Examples 2-1 to 2-26 and Comparative Examples 2-1 to 2-21 were each diluted to a concentration of 5% in dimethyl sulfoxide (DMSO), dimethyl formamide (DMF), and methylpyrrolidone (NMP), respectively, and the yellow index was measured using a ColorFlex EZ (manufacturer: HunterLab) equipment.

**Test Example 1-3: Content of metals**

The content (ppm) of metals present in each of the compositions of Examples 2-1 to 2-26 and Comparative Examples 2-1 to 2-21 was measured using Inductively Coupled Plasma Atomic Emission Spectroscopy (ICP-AES). N.D. means that the content was too low, less than 1 ppm, to be measured as a specific value.

[Table 2]

| | | Comp'n | Process conditions | Pigment residual rate (%) DMSO/DMF/NMP | Y.I. DMSO/DMF /NMP | Content of metals (ppm) (Sb/Ti/Zn) |
|---|---|---|---|---|---|---|
| Ex. 2-1 | Ex. 1-1 | 0.1 g of activated carbon | 100°C/3h | 5.4/6.1/5.7 | 1.6/1.4/1.6 | 18/5/4 |

(continued)

| | | Comp'n | Process conditions | Pigment residual rate (%) DMSO/DMF/NMP | Y.I. DMSO/DMF /NMP | Content of metals (ppm) (Sb/Ti/Zn) |
|---|---|---|---|---|---|---|
| Ex. 2-2 | Ex. 1-2 | 0.1 g of activated carbon | 100°C/3h | 6.8/6.5/6.0 | 1.4/1.5/1.3 | 15/6/3 |
| Ex. 2-3 | Ex. 1-3 | 0.1 g of activated carbon | 100°C/3h | 7.6/7.5/6.9 | 1.6/1.7/1.3 | 12/2/2 |
| Ex. 2-4 | Ex. 1-4 | 0.1 g of activated carbon | 100°C/3h | 8.1/8.3/7.8 | 1.8/1.5/1.6 | 15/4/3 |
| Ex. 2-5 | Ex. 1-5 | 0.1 g of activated carbon | 100°C/3h | 7.8/8.0/7.9 | 1.7/1.7/1.3 | 18/2/2 |
| Ex. 2-6 | Ex. 1-6 | 0.1 g of activated carbon | 100°C/3h | 6.2/6.0/6.5 | 1.5/1.2/1.0 | 16/4/2 |
| Ex. 2-7 | Ex. 1-7 | 0.1 g of activated carbon | 100°C/3h | 5.3/5.3/5.8 | 1.5/1.2/1.4 | 18/4/3 |
| Ex. 2-8 | Ex. 1-8 | 0.1 g of activated carbon | 100°C/3h | 6.5/5.5/6.1 | 1.1/1.3/1.6 | 19/2/3 |
| Ex. 2-9 | Ex. 1-9 | 0.1 g of activated carbon | 100°C/3h | 6.7/5.8/6.0 | 1.3/1.3/1.5 | 21/2/3 |
| Ex. 2-10 | Ex. 1-10 | 0.1 g of activated carbon | 100°C/3h | 7.5/6.1/6.8 | 1.4/1.3/1.6 | 20/3/3 |
| Ex. 2-11 | Ex. 1-11 | 0.1 g of activated carbon | 100°C/3h | 6.0/5.3/6.1 | 1.3/1.4/1.3 | 17/2/3 |
| Ex. 2-12 | Ex. 1-11 | 0.05 g of activated carbon | 100°C/3h | 7.9/7.1/7.8 | 1.8/1.8/1.6 | 21/2/3 |
| Ex. 2-13 | Ex. 1-11 | 0.5 g of activated carbon | 100°C/3h | 5.1/4.8/4.0 | 0.9/1.0/0.8 | 15/2/2 |
| Ex. 2-14 | Ex. 1-11 | 0.1 g of activated carbon /0.1 g of silica gel | 100°C/3h | 4.8/4.3/51 | 1.4/1.2/1.5 | N.D./N.D./ N.D. |
| Ex. 2-15 | Ex. 1-11 | 0.05 g of activated carbon /0.05 g of silica gel | 100°C/3h | 7.0/7.3/6.9 | 1.6/1.3/1.2 | N.D./N.D./ N.D. |
| Ex. 2-16 | Ex. 1-12 | 0.1 g of activated carbon | 100°C/3h | 5.6/5.3/5.8 | 1.1/1.1/1.3 | 19/4/2 |
| Ex. 2-17 | Ex. 1-13 | 0.1 g of activated carbon | 100°C/3h | 6.1/5.9/5.5 | 1.2/1.0/1.4 | 17/3/2 |
| Ex. 2-18 | Ex. 1-14 | 0.1 g of activated carbon | 100°C/3h | 8.1/7.9/7.5 | 0.9/1.1/1.4 | 15/4/3 |
| Ex. 2-19 | Ex. 1-15 | 0.1 g of activated carbon | 100°C/3h | 7.7/7.2/6.7 | 1.0/1.0/1.3 | 19/3/2 |
| Ex. 2-20 | Ex. 1-16 | 0.1 g of activated carbon | 100°C/3h | 7.4/6.9/6.1 | 1.0/1.3/1.6 | 13/3/3 |
| Ex. 2-21 | Ex. 1-17 | 0.1 g of activated carbon | 100°C/3h | 6.6/6.0/6.2 | 1.2/1.5/1.7 | 16/3/2 |
| Ex. 2-22 | Ex. 1-18 | 0.1 g of activated carbon | 100°C/3h | 7.7/7.2/6.7 | 1.7/1.0/1.2 | 15/2/4 |

(continued)

| | | Comp'n | Process conditions | Pigment residual rate (%) DMSO/DMF/NMP | Y.I. DMSO/DMF /NMP | Content of metals (ppm) (Sb/Ti/Zn) |
|---|---|---|---|---|---|---|
| Ex. 2-23 | Ex. 1-19 | 0.1 g of activated carbon | 100°C/3h | 7.7/7.2/6.7 | 1.1/1.1/1.3 | 11/3/2 |
| Ex. 2-24 | Ex. 1-1 | 0.1 g of activated carbon | 100°C/3h | 5.1/5.0/4.9 | 1.5/1.5/1.6 | 15/4/4 |
| Ex. 2-25 | Ex. 1-4 | 0.1 g of activated carbon | 100°C/3h | 8.4/8.2/7.8 | 1.7/1.6/1.6 | 17/5/3 |
| Ex. 2-26 | Ex. 1-10 | 0.1 g of activated carbon | 100°C/3h | 7.7/7.1/7.8 | 1.5/1.4/1.6 | 18/4/3 |
| C. Ex. 2-1 | C. Ex. 1-1 | 0.1 g of activated carbon | 100°C/3h | 100 | 6.1/6.4/6.2 | 121/19/38 |
| C. Ex. 2-2 | C. Ex. 1-2 | 0.1 g of activated carbon | 100°C/3h | 100 | 7.2/7.3/7.0 | 115/16/33 |
| C. Ex. 2-3 | Ex. 1-1 | - | - | 100 | 6.3/6.8/6.9 | 106/21/43 |
| C. Ex. 2-4 | Ex. 1-2 | - | - | 100 | 6.6/6.9/7.0 | 119/18/39 |
| C. Ex. 2-5 | Ex. 1-3 | - | - | 100 | 7.1/7.3/7.5 | 109/18/35 |
| C. Ex. 2-6 | Ex. 1-4 | - | - | 100 | 7.3/7.2/7.4 | 105/20/41 |
| C. Ex. 2-7 | Ex. 1-5 | - | - | 100 | 7.8/7.7/7.1 | 111/16/34 |
| C. Ex. 2-8 | Ex. 1-6 | - | - | 100 | 8.0/8.3/8.3 | 103/17/38 |
| C. Ex. 2-9 | Ex. 1-7 | - | - | 100 | 8.2/8.4/8.0 | 107/16/32 |
| C. Ex. 2-10 | Ex. 1-8 | - | - | 100 | 7.1/7.3/7.5 | 100/14/31 |
| C. Ex. 2-11 | Ex. 1-9 | - | - | 100 | 7.9/8.0/8.1 | 92/10/24 |
| C. Ex. 2-12 | Ex. 1-10 | - | - | 100 | 8.3/8.3/8.6 | 117/15/33 |
| C. Ex. 2-13 | Ex. 1-11 | - | - | 100 | *7.6/7.8/8.0* | 125/14/30 |
| C. Ex. 2-14 | Ex. 1-12 | - | - | 100 | 7.2/7.3/7.6 | 121/17/37 |
| C. Ex. 2-15 | Ex. 1-13 | - | - | 100 | 7.7/8.0/8.1 | 120/16/41 |
| C. Ex. 2-16 | Ex. 1-14 | - | - | 100 | *7.9/7.8/8.2* | 131/11/33 |
| C. Ex. 2-17 | Ex. 1-15 | - | - | 100 | 8.0/7.7/8.0 | 112/10/30 |
| C. Ex. 2-18 | Ex. 1-16 | - | - | 100 | 7.6/8.0/7.3 | 108/13/29 |

(continued)

| | | Comp'n | Process conditions | Pigment residual rate (%) DMSO/DMF/NMP | Y.I. DMSO/DMF /NMP | Content of metals (ppm) (Sb/Ti/Zn) |
|---|---|---|---|---|---|---|
| C. Ex. 2-19 | Ex. 1-17 | - | - | 100 | 7.3/7.5/7.5 | 125/11/33 |
| C. Ex. 2-20 | Ex. 1-18 | - | - | 100 | *7.7/7.6/8.0* | 116/10/39 |
| C. Ex. 2-21 | Ex. 1-19 | - | - | 100 | 7.3/7.3/7.6 | 111/16/40 |

As can be seen from Table 2 above, the purified and concentrated alcoholysis reaction compositions of Examples 2-1 to 2-26 had a very low pigment residual rate and a very low content of metals such as Sb, Ti, and Zn.

**Preparation of terephthalic acid**

**Example 3-1**

A second high-pressure reactor with a capacity of 600 ml was charged with 100 g (0.36 mole) of the purified and concentrated alcoholysis reaction composition of Example 2-2 and 200 g (11.10 moles) of water, followed by an addition thereto of 50 mg of $Zn(OAC)_2 \cdot 2H_2O$ (500 ppm relative to the total weight of the purified and concentrated alcoholysis reaction composition).

Thereafter, the temperature of the second high-pressure reactor was raised to 260°C, and a hydrolysis reaction was carried out for 4 hours while a temperature of 260°C was maintained, followed by cooling to 90°C to obtain a hydrolysis reaction product in the form of a slurry. The hydrolysis reaction product in the form of a slurry was filtered to obtain a solid, which was washed with butanol and water at 90°C and dried under vacuum to obtain 53.7 g (yield: 90%) of solid terephthalic acid (TPA).

**Example 3-2**

A second high-pressure reactor with a capacity of 600 ml was charged with 100 g (0.30 mole) of the purified and concentrated alcoholysis reaction composition of Example 2-5 and 200 g (11.10 moles) of water, followed by an addition thereto of 50 mg of $Zn(OAC)_2 \cdot 2H_2O$ (500 ppm relative to the total weight of the purified and concentrated alcoholysis reaction composition).

Thereafter, the temperature of the second high-pressure reactor was raised to 260°C, and a hydrolysis reaction was carried out for 4 hours while a temperature of 260°C was maintained, followed by cooling to 90°C to obtain a hydrolysis reaction product in the form of a slurry. The hydrolysis reaction product in the form of a slurry was filtered to obtain a solid, which was washed with pentanol and water at 90°C and dried under vacuum to obtain 43.9 g (yield: 81%) of solid terephthalic acid (TPA).

**Example 3-3**

A second high-pressure reactor with a capacity of 600 ml was charged with 100 g (0.26 mole) of the purified and concentrated alcoholysis reaction composition of Example 2-11 and 1,000 g (55.50 moles) of water, followed by an addition thereto of 50 mg of $Zn(OAC)_2 \cdot 2H_2O$ (500 ppm relative to the total weight of the purified and concentrated alcoholysis reaction composition).

Thereafter, the temperature of the second high-pressure reactor was raised to 260°C, and a hydrolysis reaction was carried out for 4 hours while a temperature of 260°C was maintained, followed by cooling to 90°C to obtain a hydrolysis reaction product in the form of a slurry. The hydrolysis reaction product in the form of a slurry was filtered to obtain a solid, which was washed with 2-ethyl-1-hexanol and water at 90°C and dried under vacuum to obtain 38.0 g (yield: 88%) of solid terephthalic acid (TPA).

**Example 3-4**

35.4 g (yield: 82%) of solid terephthalic acid (TPA) was obtained in the same manner as in Example 3-3, except that 100 g (0.26 mole) of the purified and concentrated alcoholysis reaction composition of Example 2-13 was used.

**Example 3-5**

36.7 g (yield: 85%) of solid terephthalic acid (TPA) was obtained in the same manner as in Example 3-3, except that 100 g (0.26 mole) of the purified and concentrated alcoholysis reaction composition of Example 2-14 was used.

**Example 3-6**

A second high-pressure reactor with a capacity of 600 ml was charged with 114 g (0.26 mole) of the purified and concentrated alcoholysis reaction composition of Example 2-18 and 200 g (11.10 moles) of water, followed by an addition thereto of 57 mg of $Zn(OAC)_2 \cdot 2H_2O$ (500 ppm relative to the total weight of the purified and concentrated alcoholysis reaction composition).

Thereafter, the temperature of the second high-pressure reactor was raised to 260°C, and a hydrolysis reaction was carried out for 4 hours while a temperature of 260°C was maintained, followed by cooling to 90°C to obtain a hydrolysis reaction product in the form of a slurry. The hydrolysis reaction product in the form of a slurry was filtered to obtain a solid, which was washed with decanol and water at 90°C and dried under vacuum to obtain 36.1 g (yield: 85%) of solid terephthalic acid (TPA).

**Example 3-7**

54.3 g (yield: 91%) of solid terephthalic acid (TPA) was obtained in the same manner as in Example 3-1, except that no hydrolysis catalyst was added.

**Example 3-8**

A second high-pressure reactor with a capacity of 600 ml was charged with 100 g (0.36 mole) of the purified and concentrated alcoholysis reaction composition of Example 2-2 and 200 g (11.10 moles) of water, followed by an addition thereto of 50 mg of $Zn(OAC)_2 \cdot 2H_2O$ (500 ppm relative to the total weight of the purified and concentrated alcoholysis reaction composition).

Thereafter, the temperature of the second high-pressure reactor was raised to 260°C, and a hydrolysis reaction was carried out for 4 hours while a temperature of 260°C was maintained, followed by cooling to 90°C to obtain a hydrolysis reaction product in the form of a slurry. Acetone was added to the solid obtained by filtering the hydrolysis reaction product in the form of a slurry, followed by stirring at 50°C for 4 hours. It was filtered, then washed with acetone and water at 50°C, and dried under vacuum to obtain 55.6 g (yield: 93%) of solid terephthalic acid (TPA).

**Example 3-9**

A second high-pressure reactor with a capacity of 600 ml was charged with 100 g (0.36 mole) of the purified and concentrated alcoholysis reaction composition of Example 2-2 and 200 g (11.10 moles) of water, followed by an addition thereto of 50 mg of $Zn(OAC)_2 \cdot 2H_2O$ (500 ppm relative to the total weight of the purified and concentrated alcoholysis reaction composition).

Thereafter, the temperature of the second high-pressure reactor was raised to 260°C, and a hydrolysis reaction was carried out for 4 hours while a temperature of 260°C was maintained, followed by cooling to 90°C to obtain a hydrolysis reaction product in the form of a slurry. Butanol was added to the solid obtained by filtering the hydrolysis reaction product in the form of a slurry, followed by stirring at 90°C for 4 hours. It was filtered, then washed with butanol and water at 50°C, and dried under vacuum to obtain 53.1 g (yield: 89%) of solid terephthalic acid (TPA).

**Example 3-10**

59.2 g (yield: 99%) of solid terephthalic acid (TPA) was obtained in the same manner as in Example 3-1, except that the filtrate separated through the filtration step in Example 3-1 was further used.

Specifically, the filtrate separated through the filtration step in Example 3-1 was charged to the second high-pressure reactor again, the temperature was raised to 260°C, and a hydrolysis reaction was carried out for 4 hours while a temperature of 260°C was maintained, followed by cooling to 90°C to obtain a hydrolysis reaction product in the form of a slurry. The hydrolysis reaction product in the form of a slurry was filtered to obtain a solid, which was washed with butanol and water at 90°C and dried under vacuum to obtain 5.5 g of solid terephthalic acid (TPA).

**Comparative Example 3-1**

54.4 g (yield: 91%) of solid terephthalic acid (TPA) was obtained in the same manner as in Example 3-1, except that 100 g (0.36 mole) of the composition of Comparative Example 2-4 was used.

**Comparative Example 3-2**

40.4 g (yield: 81%) of solid terephthalic acid (TPA) was obtained in the same manner as in Example 3-2, except that 100 g (0.30 mole) of the composition of Comparative Example 2-7 was used.

**Comparative Example 3-3**

36.7 g (yield: 85%) of solid terephthalic acid (TPA) was obtained in the same manner as in Example 3-3, except that 100 g (0.26 mole) of the composition of Comparative Example 2-13 was used.

**Comparative Example 3-4**

34.4 g (yield: 81%) of solid terephthalic acid (TPA) was obtained in the same manner as in Example 3-6, except that 114 g (0.26 mole) of the composition of Comparative Example 2-16 was used.

**Test Example 2-1: Pigment residual rate**

The terephthalic acid in each of Examples 3-1 to 3-10 and Comparative Examples 3-1 to 3-4 was measured for pigment residual rate (%).

Specifically, the terephthalic acid of Example 3-1 was diluted to a concentration of 5% in dimethyl sulfoxide (DMSO), dimethyl formamide (DMF), and methylpyrrolidone (NMP), respectively, and the area (B1) of an absorbance curve was obtained in 400 nm to 800 nm using a UV-vis spectrophotometer. In addition, for the composition of Comparative Example 2-4 (the composition of Example 1-2 was used in the same manner as the composition of Example 1-2 used in the preparation of terephthalic acid of Example 3-1 whereas the composition was concentrated without adding an adsorbent), the area (B2) of an absorbance curve was obtained in 400 nm to 800 nm in the same manner as above. The pigment residual rate (%) was calculated according to the following Equation B using the area of the measured absorbance curve.

[Equation B]

$$Pigment\ residual\ rate\ of\ recycled\ terephthalic\ acid\ (\%) = \frac{B1}{B2} \times 100$$

The terephthalic acid in each of Examples 3-2 to 3-10 and Comparative Examples 3-1 to 3-4 was measured for pigment residual rate (%) in the same manner as above. Specifically, as to B2 of Formula 1 above, Example 3-2 was measured using the composition of Comparative Example 2-7, Examples 3-3 to 3-5 were measured using the composition of Comparative Example 2-13, and Examples 3-6 was measured using the composition of Comparative Example 2-16. In addition, in Comparative Examples 3-1 to 3-4, B2 of Formula 1 was measured using the compositions of Comparative Examples 2-4, 2-7, 2-13, and 2-16, respectively.

**Test Example 2-2: Yellow index**

The recycled terephthalic acid in each of Examples 3-1 to 3-10 and Comparative Examples 3-1 to 3-4 was measured for yellow index (Y.I.).

Specifically, the recycled terephthalic acid in each of Examples 3-1 to 3-10 and Comparative Examples 3-1 to 3-4 was diluted to a concentration of 5% in dimethyl sulfoxide (DMSO), dimethyl formamide (DMF), and methylpyrrolidone (NMP), respectively, and the yellow index was measured using a ColorFlex EZ (manufacturer: HunterLab) equipment.

**Test Example 2-3: Content of metals**

The content (ppm) of metals present in the terephthalic acid in each of Examples 3-1 to 3-10 and Comparative Examples 3-1 to 3-10 was measured using Inductively Coupled Plasma Atomic Emission Spectroscopy (ICP-AES). N.D. means that the content was too low, less than 1 ppm, to be measured as a specific value.

**Test Example 2-4: Color-b**

The terephthalic acid in each of Examples 3-1 to 3-10 and Comparative Examples 3-1 to 3-4 was measured for color-b, a color characteristic, using a colorimeter.

[Table 3]

| | Comp'n | TPA yield (%) | Color b | Pigment residual rate (%) DMSO/DMF/NMP | Y.I. DMSO/DMF/NMP | Content of metals (ppm) (Sb/Ti/Zn) |
|---|---|---|---|---|---|---|
| Ex. 3-1 | Ex. 2-2 | 90 | 1.3 | 0/0/0 | 1.3/1.4/1.6 | N.D./N.D./N.D. |
| Ex. 3-2 | Ex. 2-5 | 81 | 1.0 | <0.5/<0.5/<0.5 | 1.1/0.9/1.0 | 1/N.D./N.D. |
| Ex. 3-3 | Ex. 2-11 | 88 | 1.3 | 0/0/0 | 1.1/1.2/1.3 | N.D./N.D./1 |
| Ex. 3-4 | Ex. 2-13 | 82 | 0.8 | 0/0/0 | 1.0/1.0/1.2 | 1/N.D./N.D. |
| Ex. 3-5 | Ex. 2-14 | 85 | 0.9 | 0/0/0 | 0.9/1.0/1.1 | N.D./N.D./N.D. |
| Ex. 3-6 | Ex. 2-18 | 85 | 1.4 | <0.5/<0.5/<0.5 | 1.2/1.2/1.1 | 2/N.D./N.D. |
| Ex. 3-7 | Ex. 2-2 | 91 | 1.2 | 0/0/0 | 1.1/1.1/1.3 | N.D./N.D./1 |
| Ex. 3-8 | Ex. 2-2 | 93 | 1.2 | 0/0/0 | 1.3/1.2/1.3 | 1/N.D./N.D. |
| Ex. 3-9 | Ex. 2-2 | 89 | 1.3 | 0/0/0 | 1.4/1.2/1.3 | N.D./N.D./N.D. |
| Ex. 3-10 | Ex. 2-2 | 99 | 1.4 | 0/0/0 | 1.6/1.5/1.7 | N.D./N.D./N.D. |
| C. Ex. 3-1 | C. Ex. 2-4 | 91 | 7.5 | 71/69/66 | 7.8/8.1/8.0 | 62/8/11 |
| C. Ex. 3-2 | C. Ex. 2-7 | 81 | 7.1 | 64/63/61 | 8.1/8.0/8.2 | 73/5/13 |
| C. Ex. 3-3 | C. Ex. 2-13 | 85 | 8.2 | 67/65/66 | 7.3/7.5/7.8 | 52/4/10 |
| C. Ex. 3-4 | C. Ex. 2-16 | 81 | 9.1 | 58/60/61 | 7.0/7.1/7.5 | 61/7/9 |

As can be seen from Table 3 above, the terephthalic acid (recycled terephthalic acid) prepared in Examples 3-1 to 3-6 not only had a low pigment retention rate and a low yellow index, but also had a very low content of metals as impurities. In particular, in Example 3-10, the filtrate, that is, the unreacted material, separated through the filtration step was used to obtain recycled terephthalic acid in a very high yield.

## Claims

1. A process for preparing terephthalic acid, which comprises:

   (1) subjecting waste polyester to alcoholysis with an alcohol having 4 or more carbon atoms to prepare a liquid composition comprising a compound represented by Formula 1; and
   (2) subjecting the liquid composition to hydrolysis:

   [Formula 1]

   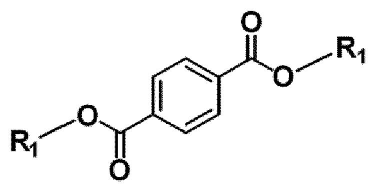

   in Formula 1, $R_1$ is an alkyl group having 4 or more carbon atoms.

2. The process for preparing terephthalic acid of claim 1, wherein the number of carbon atoms of the alcohol is 4 to 14.

3. The process for preparing terephthalic acid of claim 1, wherein the weight ratio of the waste polyester to the alcohol is 1:1 to 10.

4. The process for preparing terephthalic acid of claim 1, wherein the alcoholysis is carried out at a temperature of 160°C to 280°C and a pressure of 1 bar to 40 bar for 0.5 hour to 24 hours.

5. The process for preparing terephthalic acid of claim 1, wherein the liquid composition comprises unreacted alcohol and ethylene glycol as a by-product, and the content of the compound represented by Formula 1 in the liquid composition is 70% by mole or more.

6. The process for preparing terephthalic acid of claim 1, wherein step (1) comprises discharging the unreacted alcohol and ethylene glycol as a by-product, the alcohol is separated from the discharged mixture of alcohol and ethylene glycol, and the separated alcohol is recycled as a raw material for the alcoholysis.

7. The process for preparing terephthalic acid of claim 1, wherein step (1) comprises recovering ethylene glycol as a by-product of the alcoholysis, and the recovery rate of ethylene glycol is 65% or more.

8. The process for preparing terephthalic acid of claim 1, wherein, in step (1), an alcoholysis catalyst is added,

the alcoholysis catalyst comprises at least one cation selected from the group consisting of alkali metal ions of $Li^+$, $Na^+$, $K^+$, and $Cs^+$, alkali earth metal ions of $Be^{2+}$, $Mg^{2+}$, $Ca^{2+}$, and $Ba^{2+}$, ammonium ions of $NH^{4+}$ and $NR^{4+}$ (where R is alkyl), and $Zn^{2+}$; or at least one anion selected from the group consisting of $OH^-$, $OR^-$ (where R is alkyl), $HCO_3^-$, $CO_3^{2-}$, benzoate ion ($C_7H_5O_2^-$), 4-alkoxycarbonylbenzoate ion, acetate ion, and terephthalate ion, and
the amount of the alcoholysis catalyst added is 10 ppm to 10,000 ppm based on the total weight of the waste polyester.

9. The process for preparing terephthalic acid of claim 1, wherein, in step (1), an alcoholysis catalyst is added,

the alcoholysis catalyst comprises at least one selected from the group consisting of $Zn(OAC)_2$, $Co(OAc)_2$, $Mn(OAc)_2$, $Mg(OAc)_2$, $Ca(OAc)_2$, $Ba(OAc)_2$, LiOAc, NaOAc, KOAc, $Zn(OAC)_2{\cdot}2H_2O$, $Co(OAc)_2{\cdot}4H_2O$, $Pb(OAc)_2$, $Mn(OAc)_2{\cdot}4H_2O$, $Mg(OAc)_2{\cdot}4H_2O$, $Pd(OAc)_2$, $Ti(OBu)_4$, $Ti(OiPr)_4$, $GeO_2$, $Al(OiPr)_3$, $Na_2CO_3$, $K_2CO_3$, dibutyltin(IV) oxide, stannous octoate, titanium phosphate, and terephthalic acid, and
the amount of the alcoholysis catalyst added is 10 ppm to 10,000 ppm based on the total weight of the waste polyester.

10. The process for preparing terephthalic acid of claim 1, which further comprises purifying the liquid composition before step (2).

11. The process for preparing terephthalic acid of claim 10, wherein the purification step comprises adding at least one adsorbent selected from the group consisting of activated carbon, silica gel, alumina, zeolite, and activated clay, or adsorption through bed adsorption, and the content of the adsorbent added is 0.1% by weight to 20% by weight based on the total weight of the liquid composition.

12. The process for preparing terephthalic acid of claim 1, wherein the hydrolysis is carried out by adding water to the liquid composition at a temperature of 180°C to 280°C for 0.5 hour to 24 hours.

13. The process for preparing terephthalic acid of claim 12, wherein the weight ratio of the liquid composition to water is 1:1 to 500.

14. The process for preparing terephthalic acid of claim 1, wherein, in step (2), a hydrolysis catalyst is added,

the hydrolysis catalyst comprises at least one cation selected from the group consisting of alkali metal ions of $Li^+$, $Na^+$, $K^+$, and $Cs^+$, alkali earth metal ions of $Be^{2+}$, $Mg^{2+}$, $Ca^{2+}$, and $Ba^{2+}$, ammonium ions of $NH^{4+}$ and $NR^{4+}$ (where R is alkyl), and $Zn^{2+}$; or at least one anion selected from the group consisting of $OH^-$, $OR^-$ (where R is alkyl), $HCO_3^-$, $CO_3^{2-}$, benzoate ion ($C_7H_5O_2^-$), 4-alkoxycarbonylbenzoate ion, acetate ion, and terephthalate ion, and
the amount of the hydrolysis catalyst added is 10 ppm to 10,000 ppm based on the total weight of the liquid composition.

**15.** The process for preparing terephthalic acid of claim 1, wherein, in step (2), a hydrolysis catalyst is added,

the hydrolysis catalyst comprises at least one selected from the group consisting of $Zn(OAC)_2$, $Co(OAc)_2$, $Mn(OAc)_2$, $Mg(OAc)_2$, $Ca(OAc)_2$, $Ba(OAc)_2$, LiOAc, NaOAc, KOAc, $Zn(OAC)_2{\cdot}2H_2O$, $Co(OAc)_2{\cdot}4H_2O$, $Pb(OAc)_2$, $Mn(OAc)_2{\cdot}4H_2O$, $Mg(OAc)_2{\cdot}4H_2O$, $Pd(OAc)_2$, $Ti(OBu)_4$, $Ti(OiPr)_4$, $GeO_2$, $Al(OiPr)_3$, $Na_2CO_3$, $K_2CO_3$, dibutyltin(IV) oxide, stannous octoate, titanium phosphate, and terephthalic acid, and
the amount of the hydrolysis catalyst added is 10 ppm to 10,000 ppm based on the total weight of the liquid composition.

**Patentansprüche**

**1.** Verfahren zur Herstellung von Terephthalsäure, das Folgendes umfasst:

(1) Unterziehen von Polyesterabfall einer Alkoholyse mit einem Alkohol mit 4 oder mehr Kohlenstoffatomen, um eine flüssige Zusammensetzung herzustellen, die eine Verbindung umfasst, die durch Formel 1 dargestellt ist; und
(2) Unterziehen der flüssigen Zusammensetzung einer Hydrolyse:

[Formel 1]

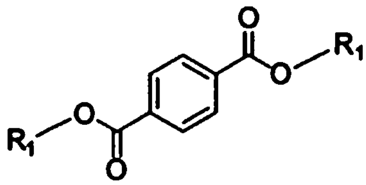

in Formel 1 ist $R_1$ eine Alkylgruppe mit 4 oder mehr Kohlenstoffatomen.

**2.** Verfahren zur Herstellung von Terephthalsäure nach Anspruch 1, wobei die Anzahl der Kohlenstoffatome des Alkohols 4 bis 14 beträgt.

**3.** Verfahren zur Herstellung von Terephthalsäure nach Anspruch 1, wobei das Gewichtsverhältnis von dem Polyesterabfall zu dem Alkohol 1:1 bis 10 beträgt.

**4.** Verfahren zur Herstellung von Terephthalsäure nach Anspruch 1, wobei die Alkoholyse bei einer Temperatur von 160 °C bis 280 °C und einem Druck von 1 bar bis 40 bar für 0,5 Stunden bis 24 Stunden durchgeführt wird.

**5.** Verfahren zur Herstellung von Terephthalsäure nach Anspruch 1, wobei die flüssige Zusammensetzung unreagierten Alkohol und Ethylenglykol als Nebenprodukt umfasst und der Gehalt der durch Formel 1 dargestellten Verbindung in der flüssigen Zusammensetzung 70 Mol-% oder mehr beträgt.

**6.** Verfahren zur Herstellung von Terephthalsäure nach Anspruch 1, wobei Schritt (1) das Abführen des nicht umgesetzten Alkohols und Ethylenglykols als Nebenprodukt umfasst, der Alkohol aus dem abgeführten Gemisch aus Alkohol und Ethylenglykol abgetrennt wird und der abgetrennte Alkohol als Rohmaterial für die Alkoholyse zurückgeführt wird.

**7.** Verfahren zur Herstellung von Terephthalsäure nach Anspruch 1, wobei Schritt (1) die Rückgewinnung von Ethylenglykol als Nebenprodukt der Alkoholyse umfasst und die Rückgewinnungsrate von Ethylenglykol 65 % oder mehr beträgt.

**8.** Verfahren zur Herstellung von Terephthalsäure nach Anspruch 1, wobei in Schritt (1) ein Alkoholyse-Katalysator zugegeben wird,

wobei der Alkoholyse-Katalysator mindestens ein Kation umfasst, das aus der Gruppe ausgewählt ist, die aus Alkalimetallionen von $Li^+$, $Na^+$, $K^+$ und $Cs^+$, Erdalkalimetallionen von $Be^{2+}$, $Mg^{2+}$, $Ca^{2+}$ und $Ba^{2+}$, Ammoniumionen von $NH^{4+}$ und $NR^{4+}$ (wobei R Alkyl ist) und $Zn^{2+}$ besteht; oder mindestens ein Anion, ausgewählt aus der

Gruppe bestehend aus $OH^-$; $OR^-$ (wobei R Alkyl ist), $HCO_3^-$, $CO_3^{2-}$, Benzoat-Ion ($C_7H_5O_2^-$), 4-Alkoxycarbonylbenzoat-Ion, Acetat-Ion und Terephthalat-Ion, und wobei die Menge des zugesetzten Alkoholyse-Katalysators 10 ppm bis 10.000 ppm beträgt, bezogen auf das Gesamtgewicht des Polyesterabfalls.

**9.** Verfahren zur Herstellung von Terephthalsäure nach Anspruch 1, wobei in Schritt (1) ein Alkoholyse-Katalysator zugegeben wird,

wobei der Alkoholyse-Katalysator mindestens einen Stoff umfasst, ausgewählt aus der Gruppe, bestehend aus $Zn(OAC)_2$, $Co(OAc)_2$, $Mn(OAc)_2$, $Mg(OAc)_2$, $Ca(OAc)_2$, $Ba(OAc)_2$, LiOAc, NaOAc, KOAc, $Zn(OAC)_2 \cdot 2H_2O$, $Co(OAc)_2 \cdot 4H_2O$, $Pb(OAc)_2$, $Mn(OAc)_2 \cdot 4H_2O$, $Mg(OAc)_2 \cdot 4H_2O$, $Pd(OAc)_2$, $Ti(OBu)_4$, $Ti(OiPr)_4$, $GeO_2$, $Al(OiPr)_3$, $Na_2CO_3$, $K_2CO_3$, Dibutylzinn(IV)-oxid, Zinnoctoat, Titanphosphat und Terephthalsäure, und
wobei die Menge des zugesetzten Alkoholyse-Katalysators 10 ppm bis 10.000 ppm beträgt, bezogen auf das Gesamtgewicht des Polyesterabfalls.

**10.** Verfahren zur Herstellung von Terephthalsäure nach Anspruch 1, das weiter das Reinigen der Flüssigkeitszusammensetzung vor Schritt (2) umfasst.

**11.** Verfahren zur Herstellung von Terephthalsäure nach Anspruch 10, wobei der Reinigungsschritt das Hinzufügen mindestens eines Adsorptionsmittels umfasst, das aus der Gruppe ausgewählt ist, die aus Aktivkohle, Silikagel, Aluminiumoxid, Zeolith und aktiviertem Ton besteht, oder eine Adsorption durch Bettadsorption, und der Gehalt des hinzugefügten Adsorptionsmittels 0,1 Gew.-% bis 20 Gew.-%, bezogen auf das Gesamtgewicht der flüssigen Zusammensetzung, beträgt.

**12.** Verfahren zur Herstellung von Terephthalsäure nach Anspruch 1, wobei die Hydrolyse durch Zugabe von Wasser zu der flüssigen Zusammensetzung bei einer Temperatur von 180 °C bis 280 °C für 0,5 Stunden bis 24 Stunden durchgeführt wird.

**13.** Verfahren zur Herstellung von Terephthalsäure nach Anspruch 12, wobei das Gewichtsverhältnis der flüssigen Zusammensetzung zu Wasser 1:1 bis 500 beträgt.

**14.** Verfahren zur Herstellung von Terephthalsäure nach Anspruch 1, wobei in Schritt (2) ein Hydrolyse-Katalysator zugegeben wird,

wobei der Hydrolyse-Katalysator mindestens ein Kation umfasst, ausgewählt aus der Gruppe, bestehend aus Alkalimetallionen von $Li^+$, $Na^+$, $K^+$ und $Cs^+$, Erdalkalimetallionen von $Be^{2+}$, $Mg^{2+}$, $Ca^{2+}$ und $Ba^{2+}$, Ammoniumionen von $NH^{4+}$ und $NR^{4+}$ (wobei R Alkyl ist) und $Zn^{2+}$; oder mindestens ein Anion, ausgewählt aus der Gruppe bestehend aus $OH^-$; $OR^-$ (wobei R Alkyl ist), $HCO_3^-$, $CO_3^{2-}$, Benzoat-Ion ($C_7H_5O_2^-$), 4-Alkoxycarbonylbenzoat-Ion, Acetat-Ion und Terephthalat-Ion, und
wobei die Menge des zugesetzten Hydrolyse-Katalysators 10 ppm bis 10.000 ppm beträgt, bezogen auf das Gesamtgewicht der flüssigen Zusammensetzung.

**15.** Verfahren zur Herstellung von Terephthalsäure nach Anspruch 1, wobei in Schritt (2) ein Hydrolyse-Katalysator zugegeben wird,

wobei der Hydrolyse-Katalysator mindestens einen Stoff umfasst, ausgewählt aus der Gruppe, bestehend aus $Zn(OAC)_2$, $Co(OAc)_2$, $Mn(OAc)_2$, $Mg(OAc)_2$, $Ca(OAc)_2$, $Ba(OAc)_2$, LiOAc, NaOAc, KOAc, $Zn(OAC)_2 \cdot 2H_2O$, $Co(OAc)_2 \cdot 4H_2O$, $Pb(OAc)_2$, $Mn(OAc)_2 \cdot 4H_2O$, $Mg(OAc)_2 \cdot 4H_2O$, $Pd(OAc)_2$, $Ti(OBu)_4$, $Ti(OiPr)_4$, $GeO_2$, $Al(OiPr)_3$, $Na_2CO_3$, $K_2CO_3$, Dibutylzinn(IV)-oxid, Zinnoctoat, Titanphosphat und Terephthalsäure, und
wobei die Menge des zugesetzten Hydrolyse-Katalysators 10 ppm bis 10.000 ppm beträgt, bezogen auf das Gesamtgewicht der flüssigen Zusammensetzung.

## Revendications

**1.** Processus de préparation d'acide téréphtalique, qui comprend :

(1) la soumission d'un déchet de polyester à une alcoolyse avec un alcool présentant 4 atomes de carbone ou

plus pour préparer une composition liquide comprenant un composé représenté par la Formule 1 ; et
(2) la soumission de la composition liquide à une hydrolyse :

[Formule 1]

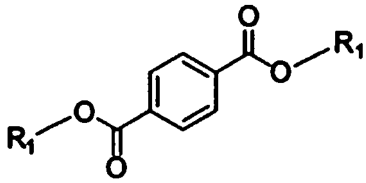

dans la Formule 1, $R_1$ est un groupe alkyle présentant 4 atomes de carbone ou plus.

**2.** Processus de préparation d'acide téréphtalique selon la revendication 1, dans lequel le nombre d'atomes de carbone de l'alcool est de 4 à 14.

**3.** Processus de préparation d'acide téréphtalique selon la revendication 1, dans lequel le rapport pondéral du déchet de polyester à l'alcool est de 1:1 à 10.

**4.** Processus de préparation d'acide téréphtalique selon la revendication 1, dans lequel l'alcoolyse est réalisée à une température de 160 °C à 280 °C et à une pression de 1 bar à 40 bar pendant 0,5 heure à 24 heures.

**5.** Processus de préparation d'acide téréphtalique selon la revendication 1, dans lequel la composition liquide comprend de l'alcool n'ayant pas réagi et de l'éthylène glycol en tant que sous-produit, et la teneur en composé représenté par la Formule 1 dans la composition liquide est supérieure ou égale à 70 % en mole.

**6.** Processus de préparation d'acide téréphtalique selon la revendication 1, dans lequel l'étape (1) comprend le rejet de l'alcool n'ayant pas réagi et de l'éthylène glycol en tant que sous-produit, l'alcool est séparé du mélange d'alcool et d'éthylène glycol rejeté, et l'alcool séparé est recyclé en tant que matière première pour l'alcoolyse.

**7.** Processus de préparation d'acide téréphtalique selon la revendication 1, dans lequel l'étape (1) comprend la récupération d'éthylène glycol en tant que sous-produit de l'alcoolyse, et le taux de récupération d'éthylène glycol est supérieur ou égal à 65 %.

**8.** Processus de préparation d'acide téréphtalique selon la revendication 1, dans lequel, à l'étape (1), un catalyseur d'alcoolyse est ajouté,

le catalyseur d'alcoolyse comprend au moins un cation sélectionné dans le groupe consistant en des ions métalliques alcalins de $Li^+$, $Na^+$, $K^+$ et $Cs^+$, des ions métalliques alcalino-terreux de $Be^{2+}$,
$Mg^{2+}$, $Ca^{2+}$ et $Ba^{2+}$, des ions ammonium de $NH^{4+}$ et $NR^{4+}$ (où R est alkyle) et $Zn^{2+}$ ; ou au moins un anion sélectionné dans le groupe consistant en $OH^-$ ; $OR^-$ (où R est alkyle),
$HCO_3^-$, $CO_3^{2-}$, un ion benzoate ($C_7H_5O_2^-$), un ion 4-alkoxycarbonylbenzoate, un ion acétate et un ion téréphtalate, et
la quantité du catalyseur d'alcoolyse ajoutée est de 10 ppm à 10 000 ppm sur la base du poids total du déchet de polyester.

**9.** Processus de préparation d'acide téréphtalique selon la revendication 1, dans lequel, à l'étape (1), un catalyseur d'alcoolyse est ajouté,

le catalyseur d'alcoolyse comprend au moins un élément sélectionné dans le groupe consistant en $Zn(OAC)_2$, $Co(OAC)_2$, $Mn(OAC)_2$, $Mg(OAC)_2$, $Ca(OAc)_2$, $Ba(OAc)_2$, LiOAc, NaOAc, KOAc, $Zn(OAC)_2 \cdot 2H_2O$, $Co(OAC)_2 \cdot 4H_2O$, $Pb(OAC)_2$, $Mn(OAc)_2 \cdot 4H_2O$, $Mg(OAc)_2 \cdot 4H_2O$, $Pd(OAc)_2$, $Ti(OBu)_4$, $Ti(OiPr)_4$, $GeO_2$, $Al(OiPr)_3$, $Na_2CO_3$, $K_2CO_3$,
oxyde de dibutylétain(IV), octoate d'étain, phosphate de titane et acide téréphtalique, et
la quantité du catalyseur d'alcoolyse ajoutée est de 10 ppm à 10 000 ppm sur la base du poids total du déchet de polyester.

**10.** Processus de préparation d'acide téréphtalique selon la revendication 1, qui comprend en outre la purification de la composition liquide avant l'étape (2).

**11.** Processus de préparation d'acide téréphtalique selon la revendication 10, dans lequel l'étape de purification comprend l'ajout d'au moins un adsorbant sélectionné dans le groupe consistant en du charbon actif, du gel de silice, de l'alumine, de la zéolite et de l'argile activée, ou une adsorption par adsorption sur lit, et la teneur en adsorbant ajouté est de 0,1 % en poids à 20 % en poids sur la base du poids total de la composition liquide.

**12.** Processus de préparation d'acide téréphtalique selon la revendication 1, dans lequel l'hydrolyse est réalisée en ajoutant de l'eau à la composition liquide à une température de 180 °C à 280 °C pendant 0,5 heure à 24 heures.

**13.** Processus de préparation d'acide téréphtalique selon la revendication 12, dans lequel le rapport pondéral de la composition liquide à l'eau est de 1:1 à 500.

**14.** Processus de préparation d'acide téréphtalique selon la revendication 1, dans lequel, à l'étape (2), un catalyseur d'hydrolyse est ajouté,

le catalyseur d'hydrolyse comprend au moins un cation sélectionné dans le groupe consistant en des ions métalliques alcalins de $Li^+$, $Na^+$, $K^+$ et $Cs^+$, des ions métalliques alcalino-terreux de $Be^{2+}$, $Mg^{2+}$, $Ca^{2+}$ et $Ba^{2+}$, des ions ammonium de $NH^{4+}$ et $NR^{4+}$ (où R est alkyle) et $Zn^{2+}$ ; ou au moins un anion sélectionné dans le groupe consistant en OH ; OR- (où R est alkyle), $HCO_3^-$, $CO_3^{2-}$, un ion benzoate ($C_7H_5O_2^-$), un ion 4-alkoxycarbonylbenzoate, un ion acétate et un ion téréphtalate, et
la quantité du catalyseur d'hydrolyse ajoutée est de 10 ppm à 10 000 ppm sur la base du poids total de la composition liquide.

**15.** Processus de préparation d'acide téréphtalique selon la revendication 1, dans lequel, à l'étape (2), un catalyseur d'hydrolyse est ajouté,

le catalyseur d'hydrolyse comprend au moins un élément sélectionné dans le groupe consistant en $Zn(OAC)_2$, $Co(OAC)_2$, $Mn(OAC)_2$, $Mg(OAC)_2$, $Ca(OAc)_2$, $Ba(OAc)_2$, LiOAc, NaOAc, KOAc, $Zn(OAC)_2 \cdot 2H_2O$, $Co(OAC)_2 \cdot 4H_2O$, $Pb(OAC)_2$, $Mn(OAc)_2$:$4H_2O$,
$Mg(OAc)_2 \cdot 4H_2O$, $Pd(OAc)_2$, $Ti(OBu)_4$, $Ti(OiPr)_4$, $GeO_2$, $Al(OiPr)_3$, $Na_2CO_3$, $K_2CO_3$, oxyde de dibutylétain(IV), octoate d'étain, phosphate de titane et acide téréphtalique, et
la quantité du catalyseur d'hydrolyse ajoutée est de 10 ppm à 10 000 ppm sur la base du poids total de la composition liquide.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 19970042469
- JP 2006083125 A